# EUROPEAN PATENT SPECIFICATION

(11) **EP 1 641 922 B1**
(45) Date of publication and mention of the grant of the patent: **26.09.2012**
(21) Application number: 04748714.5
(22) Date of filing: 07.07.2004
(51) Int. Cl.: C12N 9/12

(54) **NEWLY IDENTIFIED CHOLINEPHOSPHOTRANSFERASES AND ETHANOLAMINEPHOSPHOTRANSFERASES**
NEU IDENTIFIZIERTE CHOLINPHOSPHOTRANSFERASEN UND ETHANOLAMINPHOSPHOTRANSFERASEN
CHOLINE-PHOSPHOTRANSFERASES ET ETHANOLAMINE-PHOSPHOTRANSFERASES NOUVELLEMENT IDENTIFIEES

(30) Priority: 07.07.2003 US 485202 P; 18.12.2003 EP 03078932
(43) Date of publication of application: 05.04.2006
(73) Proprietor: Universiteit Utrecht Holding B.V., 3584 CM Utrecht (NL)
(72) Inventor: HUITEMA, Klasina, Rinsje, NL-5165 VW Waspik (NL); HOLTHUIS, Josephus, Carolus, Maria, 1011 HJ Amsterdam (NL)
(74) Representative: Jansen, Cornelis Marinus
(86) International application number: PCT/NL2004/000488
(87) International publication number: WO 2005/003336

(56) References cited:
- WO-A-03/073826
- DATABASE Geneseq [Online] 22 October 2001 (2001-10-22), "Human polypeptide SEQ ID NO 3085." XP002325874 retrieved from EBI accession no. GSP:AAM39940 Database accession no. AAM39940 -& WO 01/53312 A (HYSEQ, INC; TANG, Y., TOM; LIU, CHENGHUA; ASUNDI, VINOD; CHEN, RUI-HON) 26 July 2001 (2001-07-26)
- DATABASE Geneseq [Online] 6 September 2002 (2002-09-06), "Human cytochrome constitutive protein 45." XP002325875 retrieved from EBI accession no. GSN:ABB09578 Database accession no. ABB09578 -& WO 02/48356 A (BIOWINDOW GENE DEVELOPMENT INC. SHANGHAI; MAO, YUMIN; XIE, YI) 20 June 2002 (2002-06-20)
- DATABASE Geneseq [Online] 10 February 2003 (2003-02-10), "Human secretory polypeptide SPTM SEQ ID NO 835." XP002325876 retrieved from EBI accession no. GSN:ABP75651 Database accession no. ABP75651 -& WO 02/083876 A (INCYTE GENOMICS, INC; DAFFO, ABEL; JONES, ANISSA, L; TRAN, ALANNA-PHUN) 24 October 2002 (2002-10-24)
- DATABASE Geneseq [Online] 18 December 2003 (2003-12-18), "REMAP protein #18." XP002325877 retrieved from EBI accession no. GSN:ADC42858 Database accession no. ADC42858 -& WO 03/027228 A (INCYTE GENOMICS, INC; LAL, PREETI, G; HONCHELL, CYNTHIA, D) 3 April 2003 (2003-04-03)
- DATABASE Geneseq [Online] 1 October 2001 (2001-10-01), "Human bone marrow protein, SEQ ID NO: 346." XP002325878 retrieved from EBI accession no. GSN:AAM00870 Database accession no. AAM00870 & WO 01/53453 A (HYSEQ, INC; FORD, JOHN, E; BOYLE, BRYAN, J; TANG, Y., TOM; LIU, CHENGH) 26 July 2001 (2001-07-26)
- DATABASE EPO Proteins [Online] 2 February 2004 (2004-02-02), "Sequence 8703 from Patent WO0171042." XP002325879 retrieved from EBI accession no. EPOP:CQ580945 Database accession no. CQ580945 -& WO 01/71042 A (PE CORPORATION) 27 September 2001 (2001-09-27)
- HUITEMA KLAZIEN ET AL: "Identification of a family of animal sphingomyelin synthases." EMBO (EUROPEAN MOLECULAR BIOLOGY ORGANIZATION) JOURNAL, vol. 23, no. 1, 14 January 2004 (2004-01-14), pages 33-44, XP002325870 ISSN: 0261-4189
- DATABASE Geneseq [Online] 15 January 2004 (2004-01-15), "Human NOV13a SEQ ID 54." XP002325881 retrieved from EBI accession no. GSN:ADD49081 Database accession no. ADD49081 -& WO 03/060149 A (CURAGEN CORPORATION; GROSSE, WILLIAM, M; ALSOBROOK, II, JOHN, P; ANDER) 24 July 2003 (2003-07-24)
- DATABASE Geneseq [Online] 20 May 2004 (2004-05-20), "Human disease detection and treatment (MDDT) protein - SEQ ID 116." XP002325882 retrieved from EBI accession no. GSN:ADL22667 Database accession no. ADL22667 -& WO 03/062379 A (INCYTE GENOMICS, INC; JONES, ANISSA, L; DAHL, CHRISTOPHER, R; GIETZEN,) 31 July 2003 (2003-07-31)
- YAMAOKA SHOHEI ET AL: "Expression cloning of a human cDNA restoring sphingomyelin synthesis and cell growth in sphingomyelin synthase-defective lymphoid cells" JOURNAL OF BIOLOGICAL CHEMISTRY, vol. 279, no. 18, 30 April 2004 (2004-04-30), pages 18688-18693, XP002325871 ISSN: 0021-9258
- LUBERTO CHIARA ET AL: "Purification, characterization, and identification of a sphingomyelin synthase from Pseudomonas aeruginosa. PlcH is a multifunctional enzyme." JOURNAL OF BIOLOGICAL CHEMISTRY, vol. 278, no. 35, 29 August 2003 (2003-08-29), pages 32733-32743, XP002325872 ISSN: 0021-9258
- LUBERTO CHIARA ET AL: "Sphingomyelin synthase, a potential regulator of intracellular levels of ceramide and diacylglycerol during SV40 transformation: Does sphingomyelin synthase account for the putative phosphatidylcholine-specific phospholipase" JOURNAL OF BIOLOGICAL CHEMISTRY, vol. 273, no. 23, 5 June 1998 (1998-06-05), pages 14550-14559, XP002325873 ISSN: 0021-9258
- DATABASE UNIPROT [Online] 01 October 2002 'Testis. Homo sapiens' Retrieved from EBI, accession no. UNIPROT:Q8NHU3 Database accession no. Q8NHU3
- DATABASE UNIPROT [Online] 01 November 1996 'C. elegans cDNA YK61F1.3. F53H8.4. Caenorhabditis elegans' Retrieved from EBI, accession no. UNIPROT:Q20735 Database accession no. Q20735
- DATABASE UNIPROT [Online] 01 December 2001 'Hypothetical protein Y22D7AL.8. Caenorhabditis elegans' Retrieved from EBI, accession no. UNIPROT:Q965Q4 Database accession no. Q965Q4
- DATABASE UNIPROT [Online] 01 November 1996 'Cosmid F53B1. F53B1.2. Caenorhabditis elegans' Retrieved from EBI, accession no. UNIPROT:Q20696 Database accession no. Q20696
- DATABASE UNIPROT [Online] 01 May 2000 'W07E6.3. Caenorhabditis elegans' Retrieved from EBI, accession no. UNIPROT:Q9TYV2 Database accession no. Q9TYV2

## Description

The present invention relates to polypeptides, which transfer phosphocholine and phosphoethanolamine and the newly identification thereof, nucleotide sequences coding for any of these polypeptides and nucleotide sequences complementary thereto, plasmids, vectors and a (micro)organism or cell comprising said nucleotide sequences. Furthermore, the present invention relates to processes to produce cholinephosphotransferases and ethanolaminephosphotransferases like sphingomyelin synthase, ethanolamine phosphorylceramide synthase, phosphatidylcholine:glycoprotein cholinephosphotransferase and phosphatidylcholine:glycolipid cholinephosphotransferase. The present invention also provides the application of said nucleotide sequences to influence the equilibrium reactions or to develop compounds influencing the equilibrium reactions wherein said transferases are involved and the application of said compounds in medical use. Finally, a process has been provided to isolate candidates for functional genes of a previously unidentified enzyme from a huge database.

Cholinephospho (Ch-P) transferases and ethanolaminephosphotransferases catalyze phosphosphingolipid synthesis in animals and parasites. Sphingomyelin (SM) is an abundant constituent of cellular membranes in a wide range of organisms, from mammals (Ullman and Radin, 1974) and nematodes (Satouchi et al., 1993) to protozoa like the human malaria parasite, *Plasmodium falciparum* (Elmendorf and Haldar 1994). SM is preferentially concentrated in the outer leaflet of the plasma membrane. Its high packing density and affinity for sterols help provide a rigid barrier to the extracellular environment and play a role in the formation of lipid rafts, specialized areas in cellular membranes with important functions in signal transduction and membrane trafficking (Simons and Toomre, 2000; Holthuis et al., 2001). Since the discovery of the 'SM cycle' as a putative signaling system analogous to well-known second messenger systems like the phosphoinositide pathway, SM has emerged to the focus of interest in many research laboratories (Kolesnick and Hannun, 1999; Andrieu-Abadie and Levade, 2002).

Since the enzyme SM synthase is also able to catalyse the reverse reaction, namely the formation of PC from SM and DAG (Marggraf and Kanfer, 1984, van Helvoort et al., 1994) it may regulate, in opposite directions, the cellular levels of the bioactive lipids ceramide and DAG. The latter two are important regulators of membrane trafficking, cell proliferation and apoptosis (Kolesnic and Hannun, 1999; Bankaitis, 2002; Brose and Rosenmund, 2002, Pettus et al., 2002). Hence, one may anticipate that the physiological significance of SM synthase goes beyond the formation of SM.

The subcellular localisation of the enzyme has been the subject of numerous studies. After the initial debate had focused on whether SM synthase is located in the Golgi or at the plasma membrane (Marggraf et al.; Voelker and Kennedy, 1982; Lipsky and Pagano, 1985), subsequent work revealed evidence for the presence of SM synthase activity in both membranes (Futerman et al., 1990; Jeckel et al., 1990; van Helvoort et al., 1994). Whether SM synthesis detected at these locations is due to the presence of more than one isoform of the enzyme has remained an open issue.

Progress in understanding the biological roles of SM synthesis and its regulation is hampered by the fact that no successful purification of the responsible enzyme has been achieved. Recent work has identified a bacterial SM synthase released from *Pseudomonas aeruginosa* (Luberto et al., 2003). However, this activity is a soluble protein, hence in contrast to the animal enzyme that is tightly associated with the membrane (Ullman and Radin, 1974; Voelker and Kennedy, 1982). Other efforts focused on the isolation of SM synthase mutants by screening Chinese hamster ovary cells for resistance to a SM-directed cytolysin (Hanada et al., 1998). Instead of mutants with a primary defect in SM synthase, this method yielded mutants defective in serine palmitoyltransferase activity or blocked in ceramide transport to the site of SM synthesis (Fukasawa et al., 1999).

In the light of the prior art it is therefore highly desirable to identify and purify SM synthases, the composing amino acid sequence and microorganisms producing the same based on a process comprising the expression of nucleotide sequences coding for the amino acid sequences.

### Definitions

Throughout this application the following definitions will be used:
- homology: sharing an ancestral molecule
- identity: extent to which amino acid sequences are invariant, in other words unchanged
- "conservative" or "non-conservative" mutation: replacement of one amino acid by another, which does not change the enzyme function upon alignment (lining up sequences to achieve maximum levels of identity and conservation)
- similarity: percentage of identical amino acids or amino acids with similar properties between two sequences.

### Abbreviations

Throughout this application the following abbreviations will be used:

| | |
|---|---|
| *Human (Hu)* | |
| *Caenorhabditis elegans* or *C. elegans* (*Ce*) | |
| *Drosophila melanogaster (Dm)* | |
| *Saccharomyces cerevisiae (Sc),* | |
| *Schizosaccharomyces pombe (Sp)* | |
| *Candida albicans* (*Ca*) | |
| sphingomyelin (SM) | N-Acyl-D-sphingosine-1-phosphocholine |
| sphingomyelinase (SMase) | |
| phosphatidyl choline (PC) | 1,2-Diacyl-sn-glycero-3-phosphocholine |
| diacyl glycerol (DAG) | sn-1,2-Diacyl glycerol |
| ceramide (CER) | N-Acyl-D-sphingosine |
| inositol phosphorylceramide (IPC) | |
| lipid phosphate phosphatase (LPP) | |
| N-(7-(4-nitrobenxo-2-oxa-1,3-diazole)aminocaproylsphingosine (NBD-CER) | |
| *N*-ethylmaleimide (NEM) | |
| electrospray ionization tandem mass spectrometry (ESI-MS/MS) | |
| phytoceramide-based SMs (phytoSM) | |
| phosphorylcholine (Ch-P) | |
| cytidine-5' diphosphocholine-choline (CDP-Ch) | |
| tubovesicular membranes (TVM) | |
| phosphatidylethanolamine (PE) | |
| phosphatidylinositol (PI] | |
| phosphatidylserine (PS) | |
| phosphatidic acid (PA) | |
| phosphatidylglycerol (PG) | |
| open reading frame (ORF) | |

CSS1, CSS2 and CSS3 (for candidate SM synthase family 1-3),
-- with human CSS3α1 and CSS3α2 renamed as *hu*SMS1 and *hu*SMS2 and C. *elegans* CSS3α1*1* and C. *elegans* CSS3α2 renamed as ceSMS1 and *ceSMS2*
-- with human CSS3β renamed as *hu*SMSr (SMS related), with mice CSS3β renamed as *mo*SMS1, with C. *elegans* CSS3β as ceSMS1 and *Drosophila* CSS3β, as *do*SMS1
-- with *C*. *elegans* CSS3γ renamed as ceSMSdr, (SMS distantly related).

### Description of the figures with legends.

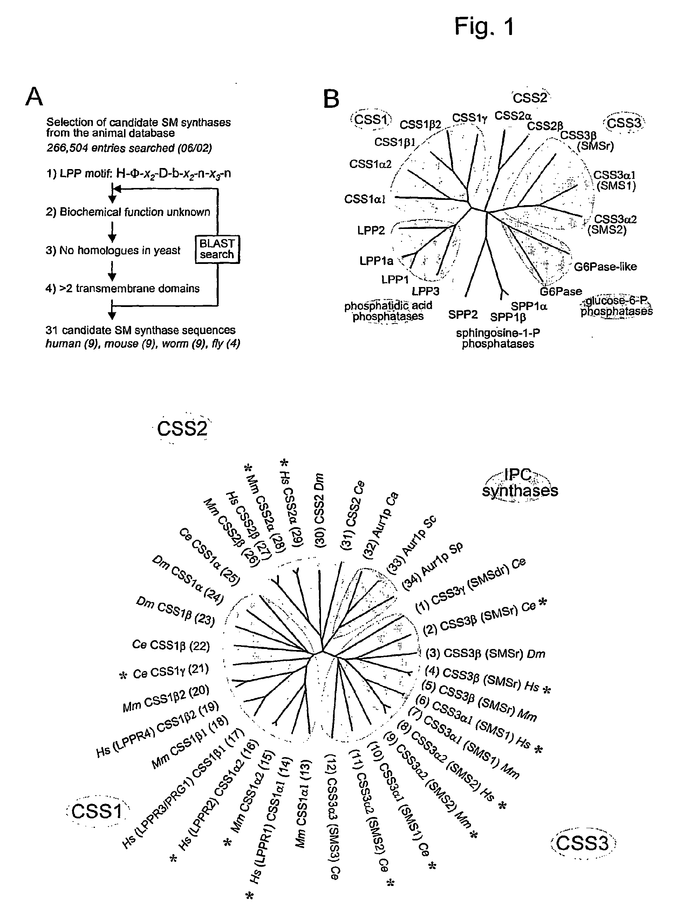

**Fig. 1**. Selection and phylogenetic analysis of candidate SM synthases.
   (A)
   Animal entries in SwissProt/TrEMBL were searched for the presence of a sequence motif shared by LPPs and Aur1p proteins and then further selected on the basis of three additional criteria, as indicated. (B) Phylogenetic tree of human candidate SM synthases (CSS) and previously characterised members of the human LPP super family. (C) Phylogenetic tree of CSS proteins from human (*Hu*), mouse (*Mm*), *Caenorhabditis elegans* (*Ce*) and *Drosophila melanogaster* (*Dm*), and of Aurlp proteins from *Saccharomyces cerevisiae* (*Sc*), *Schizosaccharomyces pombe* (*Sp*) and *Candida albicans* (*Ca*). Asterisks denote CSS proteins expressed in *S. cerevisiae* and tested for SM synthase activity. SM synthases (SMS) are marked in red. SwissProt/TrEMBL accession numbers of CSS proteins are: (1) Q9TYV2; (2) Q20696; (3) Q9VS60/Q9VS61; (4) Q96LT4; (5) Q9DA37; (6) Q86VZ5; (7) Q8VCQ6; (8) Q8NHU3; (9) Q9D4B1; (10) Q9XTV2; (11) Q20735; (12) Q965Q4; (13) Q9D606; (14) Q9NXE2; (15) Q8VCY8; (16) Q96GM1; (17) Q96MP0; (18) AAP57768; (19) Q9BQF9; (20) AAP57767; (21) Q22250; (22) Q9TXU1; (23) Q9VNT9; (24) Q9VNU1; (25) Q10022; (26) Q9D4F2; (27) Q8IY26; (28) Q91WB2; (29) Q96SS7; (30) Q8T8T9; (31) Q22461. Note that (4) Q96LT4 contains a partial protein sequence and that the complete ORF was deduced from a corresponding EST clone (see the Table).

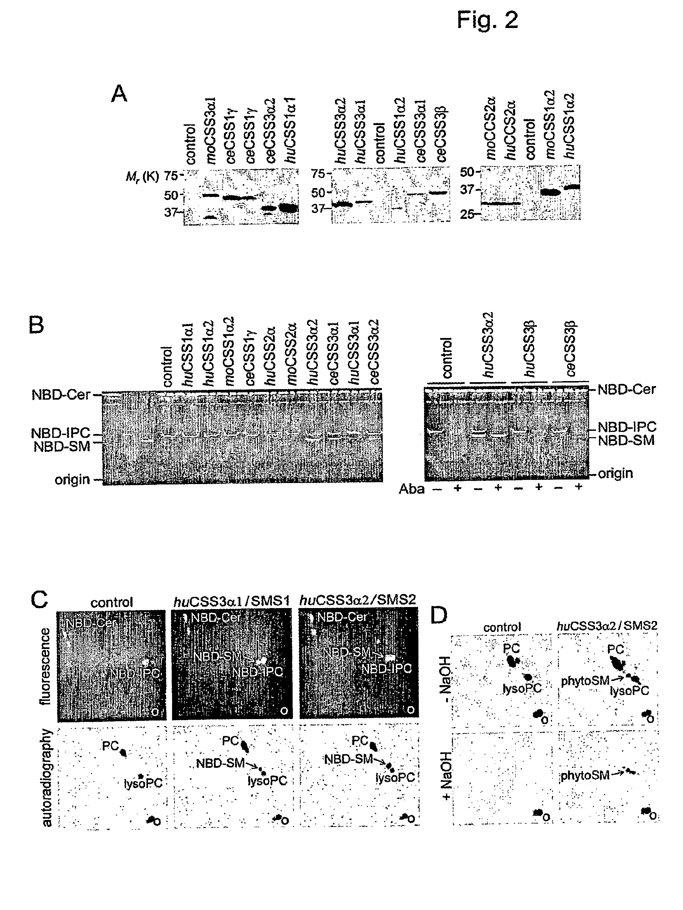

**Fig. 2**. A subset of CSS3 family members displays SM synthase activity upon expression in yeast. (A) Immunoblots of cells expressing various CSS proteins were stained with antibodies recognising the V5 epitope-tagged carboxy termini of CSS proteins. Control denotes cells transformed with empty vector. (B) TLC separation of reaction products generated when NBD-ceramide (NBD-Cer) was incubated with lysates of control or CSS-expressing cells in the presence (+) or absence (-) of IPC synthase inhibitor, aureobasidin A (Aba). (C) Metabolic labelling of cells expressing human CSS3α1/SMS1 or CSS3α2/SMS2 with [¹⁴C]-choline and NBD-ceramide. The lipids were extracted, separated by two-dimensional TLC and analysed for fluorescence and radioactivity. Note that SMS1- and SMS2-expressing cells, but not control cells, synthesized NBD-SM, and that this NBD-SM was labelled with [¹⁴C]-choline. (D) Metabolic labelling of cells expressing human CSS3α2/SMS2 with [¹⁴C]-choline. The lipids were extracted, deacylated by mild alkaline hydrolysis (+NaOH) or control incubated (-NaOH) and separated by two-dimensional TLC before autoradiography. Note that SMS2-expressing cells synthesized alkaline-resistant species of [¹⁴C]-choline-labelled lipids (phytoSM) that were absent in control cells.

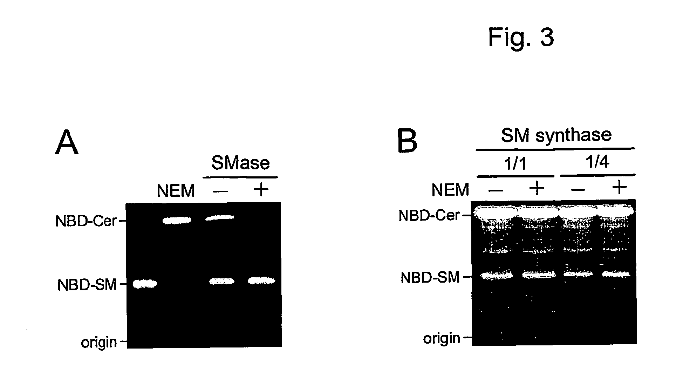

**Fig. 3**. NEM inhibits yeast-associated SMase but not mammalian SM synthase. (A) TLC separation of reaction products generated when NBD-SM (1.8 µM) was incubated with lysates of exponentially grown yeast cells (20 OD₆₀₀cells) in the presence (+) or absence (-) of N-ethylmaleimide (NEM; 1 mM). Reactions were in 0.35 ml RB1 buffer containing 1 µg/ml of IPC synthase inhibitor (aureobasidin A) and incubated for 30 min at 37 ° C. (B) TLC separation of reaction products generated when NBD-ceramide (NBD-Cer; 1,8 mM) was incubated with lysates of HeLa cells (1/1: 2 x 10⁶ cells; 1/4: 0.5 x 10⁶ cells) in the presence (+) or absence (-) of NEM (1 mM). Reactions were in 0.35 ml PNS buffer, and incubated for 30 min at 37°C. Lipid extraction and TLC analysis were as described in Materials and methods.

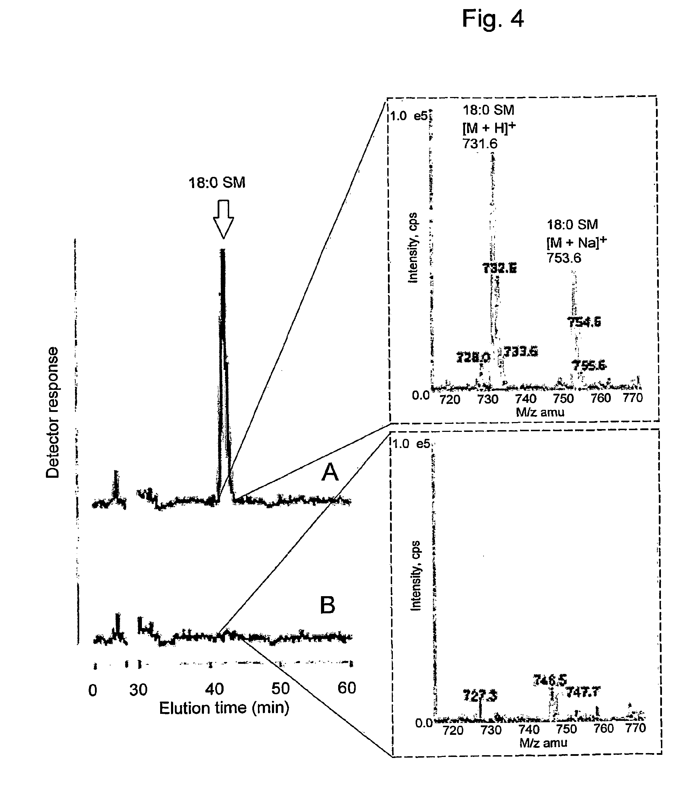

**Fig. 4****.** Detergent extracts of human CSS3α2/SMS2-expressing yeast cells support SM formation from bovine brain ceramides. Lines **A** and **B** show the reconstituted ion chromatograms of m/z 731.6, corresponding to the m/z ratio of protonated 18:0 SM, during the separation of molecular species of choline-containing phospholipids after solid phase extraction of membrane extracts that had been incubated with bovine brain ceramides and egg PC. The elution time of an authentic standard of 18:0 SM is indicated in the chromatogram by an arrow (42.3 minutes). Line **A** was derived from detergent extracts of human CSS3α2/SMS2-expressing cells and line **B** from detergent extracts of control cells. Mass spectra recorded at the elution time of 18:0 SM confirmed that formation of 18:0 SM occurred in CSS3α2/SMS2-containing extracts (top right panel), but not in control extracts (bottom right panel).

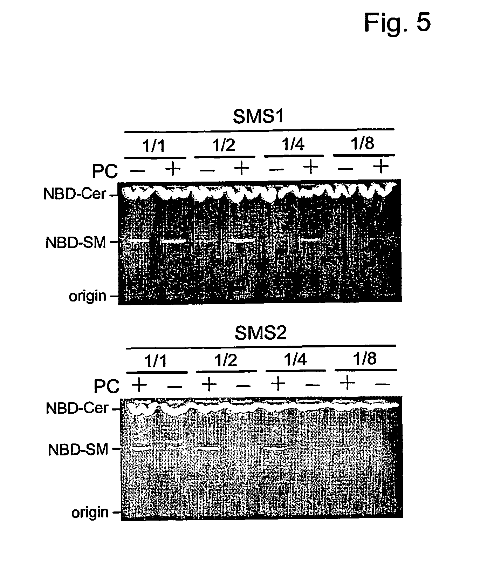

**Fig. 5****.** SM synthase activity in detergent extracts of human SMS1- or SMS2-expressing yeast cells is stimulated by externally added PC. Detergent extracts of yeast cells expressing human SMS1 or SMS2 were diluted 2- to 8-fold in extraction buffer, and then incubated with NBD-ceramide (1.8 µM) in the presence (+) or absence (-) of externally added PC (22 µM), as described in Materials and methods. Formation of NBD-SM was monitored by one-dimensional TLC.

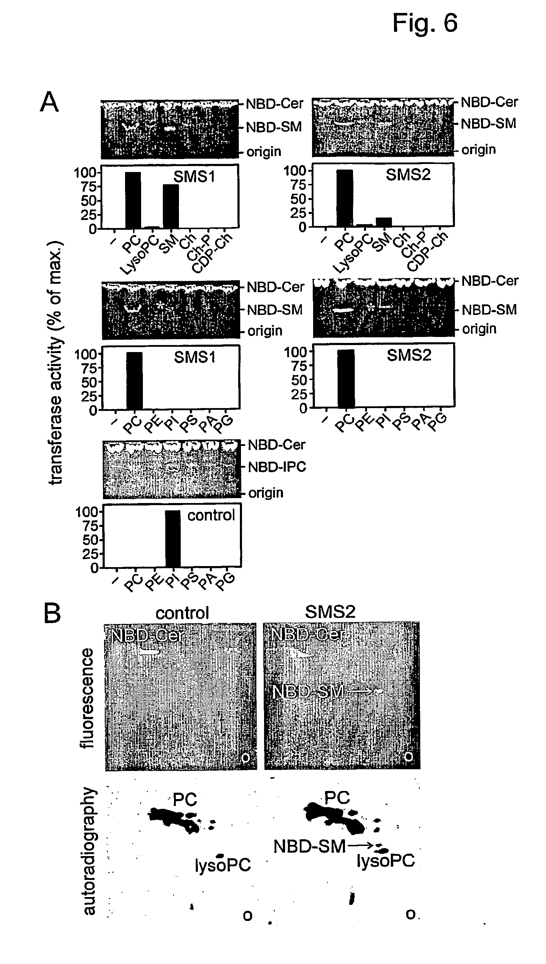

**Fig. 6****.** Human SMS1 and SMS2 function as PC:ceramide choline phosphotransferases. (A) Detergent extracts of yeast cells expressing human SMS1, SMS2 or transformed with empty vector (control) were incubated with NBD-ceramide (1.8 µM) in the presence or absence of different potential head group donors (22 µM), as indicated. Formation of NBD-SM or NBD-IPC was monitored by one-dimensional TLC and quantified as described in Materials and methods. Note that addition of PI stimulated formation of NBD-IPC in all three extracts (asterisks). PC, phosphatidylcholine; SM, sphingomyelin; Ch, choline; Ch-P, phosphorylcholine; CDP-Ch, cytidine-5' diphosphocholine; PE, phosphatidylethanolamine; PI, phosphatidylinositol; PS, phosphatidylserine; PA, phosphatidic acid; PG, phosphatidylglycerol. (**B**) Detergent extracts of SMS2-expressing and control cells were incubated with NBD-ceramide and [³H]-choline labelled PC. The lipids were extracted, separated by two-dimensional TLC and analysed for fluorescence and radioactivity. Note that only SMS2-expressing cells synthesized NBD-SM, and that this NBD-SM was labelled with [³H]-choline.

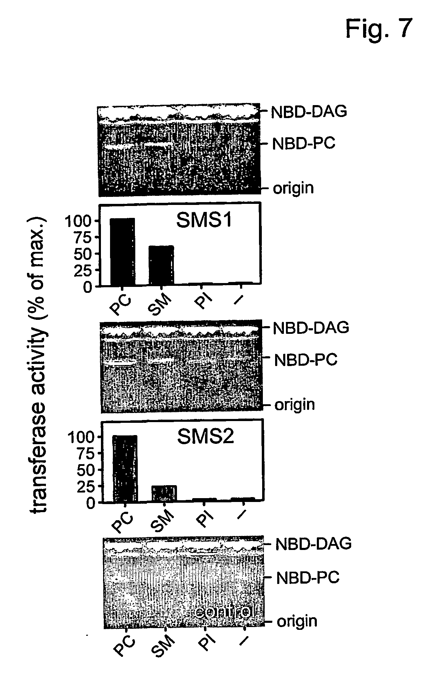

**Fig. 7****.** Human SMS1 and SMS2 exhibit reverse activity. Detergent extracts of yeast cells expressing human SMS1, SMS2 or transformed with empty vector (control) were incubated with NBD-diacylglycerol (NBD-DAG; 1.8 µM in the presence or absence of different head group donors (22 µM), as indicated. Formation of NBD-PC was monitored by one-dimensional TLC and quantified as described in Materials and methods.

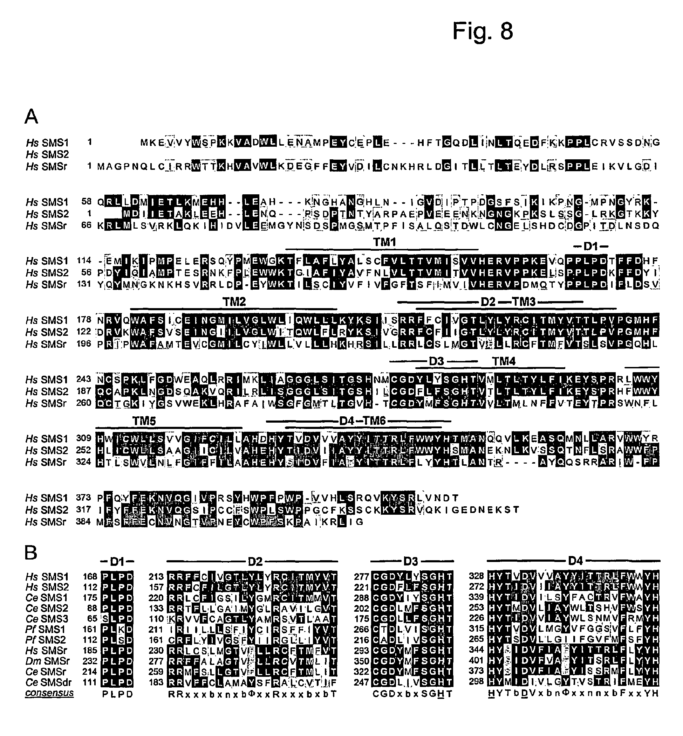

**Fig. 8****.** Conserved sequence motifs in SMS1, SMS2 and SMSr proteins. (**A**) Alignment of human SMS1, SMS2 and SMSr amino acid sequences. Identical residues are highlighted in black and conservative amino acid substitutions in grey. Conserved residues within four homology motifs, designated D1 to D4, are highlighted in blue and conservative amino acid substitutions in the D2 and D4 motifs of SMSr proteins in green. Note that motifs D3 and D4 display similarity to the C2 and C3 domains in LPPs, with identical residues highlighted in red. Regions predicted to form transmembrane domains, TM1 to TM6, are marked by a black line. (**B**) Alignment of the four homology motifs in SMS and SMSr proteins from humans (*Hu*), *C. elegans* (*Ce*), *P. falciparum* (*Pf*) and *D. melanogaster* (*Dm*). PlasmoDB accession no. of *Pf*SMS1 and *Pf*SMS2 are MAL6P1.177 and MAL6P1.178, respectively. Putative active site residues in consensus sequences are underlined. Symbols used are n, small neutral amino acid; Φ, aromatic amino acid; b, branched amino acid; x, any amino acid.
**Fig. 9**. Sequence listings 1-11: conserved sequence motifs of phosphotransferases as indicated in figure 8 B
   Sequence listings 12 - 22: polypeptide sequences of cholinephosphotransferases and ethanolaminephosphotransferases
   - 12.: *human* sphingomyelin synthase 1
   - 13.: *human* sphingomyelin synthase 2
   - 14.: *human* sphingomyelin synthase related
   - 15.: *Caenorhabditis elegans* sphingomyelin synthase 1
   - 16.: *Caercorhabditis elegans* sphingomyelin synthase 2
   - 17.: *Caenorhabditis elegans* sphingomyelin synthase 3
   - 18.: *Caenorhabditis elegans* sphingomyelin synthase distantly related
   - 19.: *Caenorhabditis elegans* sphingomyelin synthase related
   - 20.: *Plasmodium falciparum* sphingomyelin synthase 1
   - 21.: *Plasmodium falciparum* sphingomyelin synthase 2
   - 22.: *Drosophila melanogaster* sphingomyelin synthase related.
**Fig. 10****.** Human SMS1 and SMS2 are encoded by ubiquitously expressed genes. Northern blot analysis of SMS1 and SMS2 transcripts (arrows) in various human tissues. Random prime-labelled human SMS1 or SMS2 cDNA was hybridised to a human poly(A)⁺ RNA blot (Origene, Rockville, MD). As a control for loading, the RNA blot was stripped and rehybridized with a human ß-actin cDNA probe. Mobilities of RNA size markers are indicated.
**Fig. 11****.** Human SMS1 and SMS2 localise to different cellular organelles. (**A**)
   HeLa cells co-transfected with V5-tagged SMS1 and *myc*-tagged sialyltransferase were incubated in the presence or absence of 10 µM nocodazole for 60 min at 37°C, fixed, and then co-stained with rabbit anti-V5 and mouse anti-*myc* antibodies. Counterstaining was with FITC-conjugated goat anti-rabbit and Texas red-conjugated goat anti-mouse antibodies. (**B**) HeLa cells transfected with V5-tagged SMS2 were biotinylated on ice, fixed and then co-stained with mouse anti-V5 and rabbit anti-biotin antibodies. Counterstaining was with Texas red-conjugated goat-anti mouse and FITC-conjugated goat anti-rabbit antibodies. Bar, 10 µm.

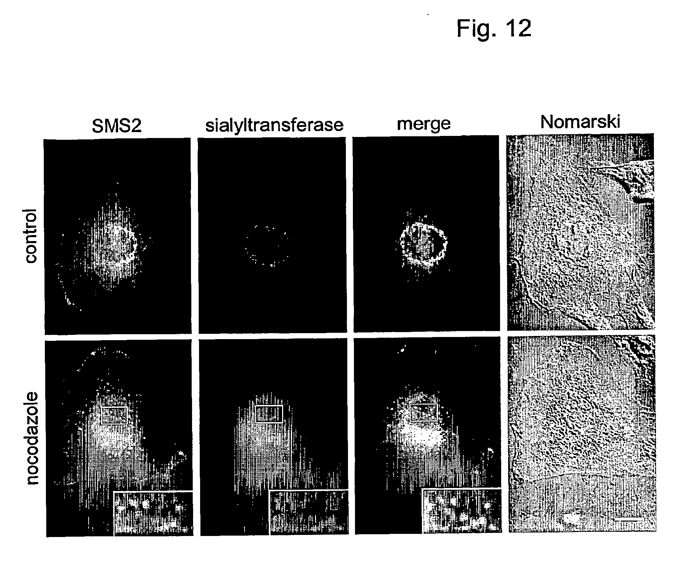

**Fig.12****.** Human SMS2 is partially associated with the Golgi. HeLa cells co-transfected with V5-tagged SMS2 and myc-tagged sialyltransferase were incubated in the presence or absence of 10 µM nocodazole for 60 min at 37°C, fixed, and then co-stained with rabbit anti-V5 and mouse anti-myc antibodies. Counterstaining was with FITC-conjugated goat anti-rabbit and Texas red-conjugated goat-anti-mouse antibodies. Bar, 10 µm.

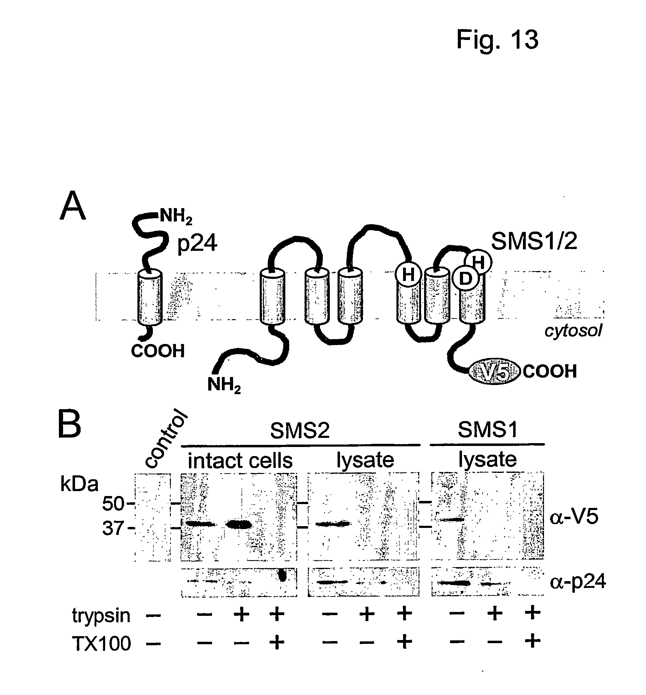

**Fig. 13**. Membrane topology of human SMS1 and SMS2 proteins. (A) Schematic
   view of the predicted membrane topologies of V5-tagged SMS1 and SMS2, and the Golgi-associated type I membrane protein, p24. (B) Immunoblot of intact or lysed HeLa cells expressing V5-tagged SMS1 or SMS2 and pretreated with 8 mM trypsin for 30 min at 30 °C in the presence or absence of 0.4% Triton X100, as indicated. Immunoblots were stained with antibodies against the V5 epitope (α-V5) or against p24 (α -p24).

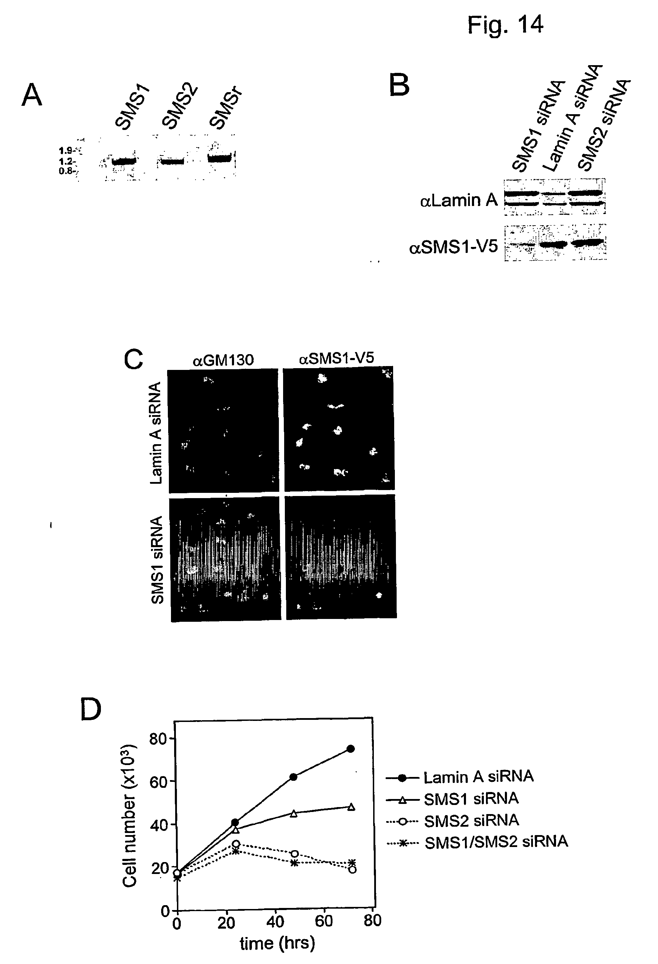

**Fig. 14****.** SMS1 and SMS2 are required for cell growth and survival. (A) SMS1, SMS2 and SMSr are expressed in human HeLa cells. Open reading frames of human SMS1, SMS2 and SMSr were amplified by reverse-transcriptase PCR on 5 µg total RNA isolated from HeLa cells, according to instructions of the manufacturer (Titan One Tube RT-PCR, cat. No. 1855476, Roche Diagnostics GmbH, Mannhein, Germany). Products were separated on a 1% agarose gel and stained with ethidium bromide. (B) HeLa cells stably transfected with V5-tagged SMS1 (SMS1-V5/pcDNA3.1) were treated with lamin A, SMS1 or SMS2 siRNA duplexes for 72 hr before Western blot analysis using anti-V5 and anti-lamin A antibodies, as described (Elbashir et al., Nature 411, 494-498). Lamin A, SMS1 and SMS2 were targeted with siRNA duplexes designed from the sequences 5'-aactggacttccagaagaacatc-3', 5'-aactacactcccagtacctgg-3', and 5'-aacccaagagcttatccagtg-3', respectively. (C) Hela cells stably transfected with V5-tagged SMS1 were treated with lamin A or SMS1 siRNA for 72 hr, and then fixed, permeabilized and co-stained with mouse-anti GM130 and rabbit anti-V5 antibodies. Counterstaining was with Texas red-conjugated goat-anti-mouse and FITC-conjugated goat-anti-rabbit antibodies. (D) Growth curves of Hela cells stably transfected with V5-tagged SMS1 and treated with lamin A, SMS1 and/or SMS2 siRNA duplexes in serum-free Optimem I medium supplemented with Glutamax I (GIBCO/Invitrogen, Carlsbad, CA). Cell numbers were assessed at 0, 24, 48 and 72 hr after treatment with siRNA duplexes.

| **Table:** EST clones and primers used in this study | | | |
|---|---|---|---|
| Protein name | Accession number¹ | EST clone ID | Primer pairs N- and C-terminal (5'-3') |
| *Hu*CSS1α1 | Q9NXE2 | IRAKp961F14 32² | AAGCCATGGCTGTAGGAAACAACAC GGTAACTTCGGTCATGGAC |
| *Hu*CSS1α2 | Q96GM1 | IRALp962F05 | AAGCCATGGCGGGAGGGAGA |
| | | 26² | GGTGGCCACGGCGGGC |
| *Hu*CSS2α | Q96SS7 | IRALp962I04 17² | AAGCCATGCCAGCTTCCCAGAG CCAGGCAGAGATGAGCATC |
| *Hu*CSS3α1 | Q86VZ5 | LGN01891³ | AAGCCATGATCCTTGTAGGACTCTG TGTGTCATTCACCAGCCGG |
| (*SMS1*) | | | |
| *Hu*CSS3α2 | Q8NHU3 | IRAKp961F14 33² | AAGCCATGAATATGTTTGATGCTGACA ATCCTTATAAGCCCGTGTGG |
| (*SMS2*) | | | |
| *Hu*CSS3β | Q96LT4 | IMAGp958I13 1239Q² | AAGCCATGGCAGGTCCTAATCAACTC TCCAATTAGTCTTTTCATTATTGC |
| *Mm*CSS1α2 | Q8VCY8 | IRAKp961L15 31² | AAGCCATGGCTGGAGGGAG GGTGGCCACGGCGGGC |
| *Mm*CSS2α | Q91WB2 | IMAGp998B0 810812² | AAGCCATGCCAGCTTCCCAGA CCAGGCAGAGATGAGCATC |
| *Mm*CSS3α1 | Q8VCQ6 | IRAKp961L21 41² | AAGCCATGAAGGAAGTGGTTTAC TGTGTCGTTTACCAGCCGG |
| *(SMS1)* | | | |
| *Ce*CSS1γ | Q22250 | YK289g7 /YK572h5⁴ | AAGCCATGTCCGTGCCAGCTTCG GTACCGATATCCATCATTTTG |
| *Ce*CSS3α1 | Q9XTV2 | YK559h9/ YK517e10⁴ | AAGCCATGAAAATGTCTTGGAATCATCA |
| *(SMS1)* | | | |
| *Ce*CSS3α2 | Q20735 | YK428e6/ | AAGCCATGACAAACAGTTCGGAGTTC TTGCAATTTGTAGTTGATACGA |
| *(SMS2)* | | YK109h8⁴ | |
| *Ce*CSS3β | Q20696 | YK524b8⁴ | AAGCCATGCTGGATAACAGACCTATAC ATTGTGCTTTTTGGTATGATTTTTG |

| | | | |
|---|---|---|---|
| 1 Swiss-Prot/TrEMBL 2 Obtained from RZPD Deutsches Ressourcenzentrum für Genomforschung, Berlin, Germany 3 Provided by Dr. Sumio Sugano, Institute of Medical Science, University of Tokyo, Japan 4 Provided by Dr. Yuji Kohara, National Institute of Genetics, Mishima, Japan | | | |

To achieve the targets of the invention a complementary approach was pursued for the identification of animal SM synthase that takes advantage of structural information available for enzymes catalyzing analogous reactions. In contrast to most animal cells, plants, fungi and yeast do not produce SM. Instead, these organisms add phosphoinositol to phytoceramide to generate inositol phosphorylceramide, or IPC (Dickson, 1998). IPC production in the yeast *Saccharomyces cerevisiae* requires the product of the *AUR1* gene (Nagiec et al., 1997).

Sequence analysis of Aur1p proteins from different fungi revealed four conserved motifs (Heidler and Radding, 2000) two of which are similar to the C2 and C3 domains present in members of the lipid phosphate phosphatase (LPP) family (Waggoner et al., 1999).

LPPs play a critical role in cell signaling by controlling the conversion of bioactive lipid phosphate esters such as (lyso)phosphatidic acid and sphingosine-1-phosphate to their dephosphorylated counterparts. The conserved C1, C2 and C3 domains in LPPs likely constitute the active site for cleavage of the bond between the lipid hydroxyl and phosphate groups (Neuwald, 1997).
In the case of Aur1p, this reaction would represent the first step in the transfer of inositol phosphate from PI, with the resulting enzyme-phosphate intermediate being subjected to nucleophilic attack by the oxygen of ceramide rather than the oxygen of water used by LPPs. The presence of LPP-like motifs, together with the IPC synthase activity found associated with affinity-purified Aur1p, suggests that Aurlp is directly responsible for IPC synthesis.

Insects and mammals contain an ethanolamine phosphorylceramide (EPC) synthase, another phosphotransferase, that may be structurally related to SM synthase.

The insect *Drosophila melanogaster* (*Dm*) does not generate SM, but instead produces phosphoethanolamine phosphorylceramide (EPC (Rietveld et al., (17). Moreover, it has been shown that the endoplasmic reticulum of mammalian cells harbours an EPC synthase (Malgat et al., 1987). Furthermore, the nematode *Caenorhabdatis elegans* (*Ce*) generates phosphorylcholine-substituted glycolipids (Lochnit, G., et al., 2000) still another phosphotransferase, that may be structurally related to SM synthase.

Based on the above considerations a bioinformatics and functional cloning strategy was developed to identify the enzyme responsible for SM synthesis in animals and to identify other cholinephosphotransferases. Described is a surprisingly successful strategy which comprises a process to isolate candidates for functional genes of a previously unidentified enzyme with known activity from a huge database by combining at least four characteristics based on data from bio-informatics and from biochemistry, viz.
- presence of a sequence motif shared with previously identified enzymes having a related function
- biochemical function of the gene should be unknown until now
- no structural homologues in an organism that does not contain the enzyme
- ability to mediate a reaction catalysed by the unidentified enzyme upon its heterologous expression in an organism or cell lacking said enzyme activity. Preferably also the presence or non-presence of transmembrane domains depending on the working mechanism of the enzyme in relation to the membrane is considered.

By searching the human, mouse, *C. elegans* and *Drosophilia* protein databases using a sequence motif shared by LPPs and Aur1p homologues and further selection on the basis of the three additional criteria mentioned above and, a set of cholinephosphotransferase sequences was collected as depicted in Fig. 8B. Conserved residues within a lot of homology motifs have been found.

Therefore, the present invention further provides an isolated polypeptide with sphingomyelin synthase activity, ethanolamine phosphorylceramide synthase activity, phosphatidylcholine:glycoprotein cholinephosphotransferase activity or phosphatidylcholine:glycolipid cholinephosphotransferase activity, said polypeptide comprising the amino acid motifs:
(a) P-L-X-D-X(35-75)-R-R-X(8)-[YF]-X(2)-R-X(6)-T;
(b) C-X-D-X(3)-S-G-H-T; and
(c) H-Y-[TS]-X-D-[VI] -X(3)-[FYI]-X(6)-F-X(2)-Y-H,
whereby the polypeptide does not comprise an amino acid sequence selected from SEQ ID No 12, 13, 15, 16, 19, 21 and 22 as depicted in Figure 9.

Peptide sequences comprising the indicated amino acid motifs (a), (b) and (c) have been described in, for example, WO01/53312, WO02/48356 WO02/083876, WO03/027338; WO01/53453 and WO01/71042. However, these documents do not describe that the peptides have sphingomyelin synthase activity, ethanolamine phosphorylceramide synthase activity, phosphatidylcholine:glycoprotein cholinephosphotransferase activity or phosphatidylcholine: glycolipid cholinephosphotransferase activity.

The wording isolated means that said polypeptide has been identified in a database comprising a large amount of polypeptides.

Furthermore the present invention provides a polypeptide comprising an amino acid sequence with at least 90% similarity to any of the SEQ ID No. 14, 17, 18 and 20 as depicted in Figure 9.

Furthermore, a nucleotide sequence selected from the group consisting of a nucleotide sequence coding for any of the amino acid sequences of a polypeptide according to the invention with one or more of said activities and an anti sense nucleotide sequence that is complementary thereto is provided, together with a plasmid, a vector and an organism or micro organism and a cell line comprising said nucleotide sequences.

According to a further preferred embodiment of the present invention a process is provided for producing sphingomyelin synthase or sphingomyelin comprising the expression of the nucleotide sequences coding for a polypeptide with sphingomyelin synthase activity in a (micro)organism or cell line comprising the nucleotide sequences coding for such a polypeptide. Furthermore, the use of one or more of the nucleotide sequences coding for a polypeptide with sphingomyelin synthase activity to influence the reaction

CER + PC ↔ SM + DAG

*in vitro* has been provided. In this way a powerful means has been created to enhance the conversion of CER and PC into SM and DAG and vice versa. Besides this, the present invention also provides the use of one or more of the nucleotide sequences coding for a polypeptide comprising the amino acid motifs (a), (b) and (c), with sphingomyelin synthase activity to identify or develop compounds influencing the reaction

CER + PC ↔ SM + DAG.

According to still a further preferred embodiment of the present invention a process is provided for producing ethanolamine phosphorylceramide synthase or ethanolamine phosphorylceramide comprising the expression of the nucleotide sequences coding for a polypeptide comprising the amino acid motifs (a), (b) and (c), with ethanolamine phosphorylceramide synthase activity in a (micro)organism or cell line comprising the nucleotide sequences coding for such a polypeptide. Furthermore, the use of one or more of the nucleotide sequences coding for a polypeptide comprising the amino acid motifs (a), (b) and (c), with ethanolamine phosphorylceramide activity to influence the reaction

CER + PE ↔ EPC + DAG

*in vitro* has been provided. In this way a powerful means has been created to enhance the conversion of CER and PE into EPC and DAG and vice versa. Besides this, the present invention also provides the use of one or more of the nucleotide sequences coding for a polypeptide comprising the amino acid motifs (a), (b) and (c), with ethanolamine phosphorylceramide synthase activity to identify or develop compounds influencing the reaction

CER + PE ↔ EPC + DAG.

According to another preferred embodiment of the invention the application of the compounds mentioned above which can be identified or developed by the use of one or more of the nucleotide sequences coding for a polypeptide with sphingomyelin synthase and/or ethanolamine phosphorylceramide synthase in medical use, especially for the manufacture of medicaments treating a disease selected from the group consisting of cancer, metabolic diseases, for instance the Niemann Pick A disease and diseases caused by parasites, for instance malaria, is provided.

According to a further preferred embodiment of the present invention a process is provided for producing phosphatidyl:glycoprotein cholinephosphotransferase or phosphatidyl:glycolipid cholinephosphotransferase, phosphorylcholine-substituted glycoprotein or phosphorylcholine-substituted glycolipid comprising the expression of the nucleotide sequences coding for a polypeptide comprising the amino acid motifs (a), (b) and (c), with phosphatidyl:glycoprotein cholinephosphotransferase or phosphatidyl:glycolipid cholinephosphotransferase activity in a (micro)organism or cell line comprising the nucleotide sequences coding for the corresponding polypeptide.
"Corresponding" stands for the nucleotide sequences which code for the enzyme with the corresponding activity, so either for the enzyme with the phosphatidyl:glycoprotein cholinephosphotransferase activity or for the enzyme with the phosphatidyl:glycolipid cholinephosphotransferase activity. Furthermore, the use of one or more of the nucleotide sequences coding for a polypeptide comprising the amino acid motifs (a), (b) and (c), with phosphatidyl:glycoprotein cholinephosphotransferase or phosphatidyl:glycolipid cholinephosphotransferase activity to influence the reaction

glycolipid/protein + PC ↔ PC-substituted glycolipid/protein + DAG

created to enhance the conversion of glycolipid/protein and PC into PC-substituted glycolipid/protein and DAG and vice versa.

Besides this, the present invention also provides the use of one or more of the nucleotide sequences coding for a polypeptide comprising the amino acid motifs (a), (b) and (c), with phosphatidyl:glycoprotein cholinephosphotransferase activity or phosphatidyl:glycolipid cholinephosphotransferase activity to identify or develop compounds influencing the reaction

glycolipid/protein + PC ↔ PC-substituted glycolipid/protein + DAG.

According to another preferred embodiment of the invention the application of the compounds mentioned above which can be identified or developed by the use of one or more of the nucleotide sequences coding for a polypeptide comprising the amino acid motifs (a), (b) and (c), with phosphatidyl:glycoprotein cholinephosphotransferase or phosphatidyl:glycolipid cholinephosphotransferase in medical use, especially for the manufacture of a medicament treating a disease caused by parasitic nematodes, is provided.

A detailed description of the various aspects of the present invention will be given here below.

A novel family of integral membrane proteins likely responsible for SM synthesis in animal cells was identified according to the process of the present invention. The two human members of this family, SMS1 and SMS2, were subjected to a detailed biochemical analysis and it is believed that their functional assignment as SM synthases is justified on the following grounds.

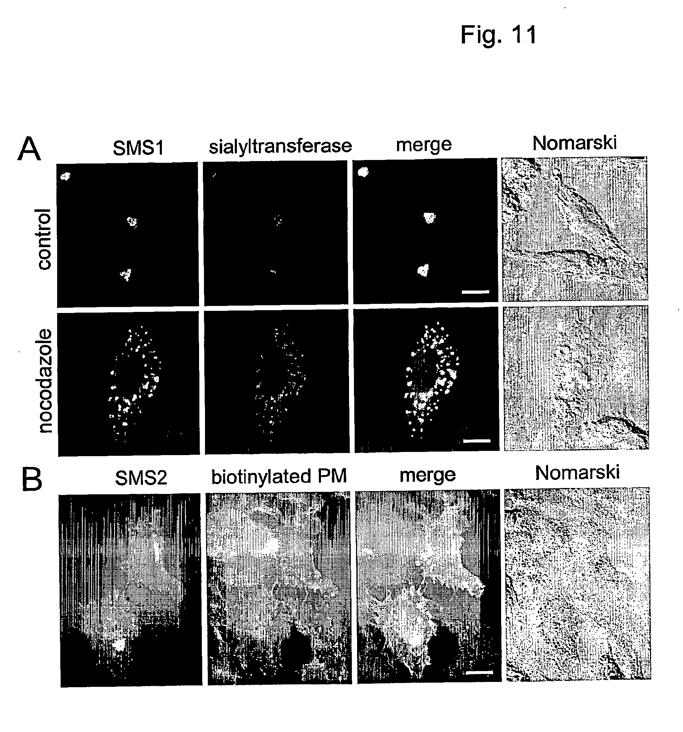

First, heterologous expression of SMS1 or SMS2 proves sufficient to support SM synthesis in the yeast *S. cerevisiae,* an organism lacking endogenous SM synthase activity (figures 2, 4 and 6). Second, in keeping with the enzymatic properties of mammalian SM synthase reported in the literature, SMS1 and SMS2 function as bi-directional lipid cholinephosphotransferases capable of converting PC and ceramide to SM and DAG and vice versa (figures 6 and 7). Third, SMS1 and SMS2 localize to cellular organelles previously established as the principal sites of SM synthesis, namely the Golgi (SMS1) and the plasma membrane (SMS2), figure 11. Fourth, SMS1 and SMS2 share sequence motifs containing putative active site residues with lipid phosphate phosphatases and candidate PI:ceramide inositolphosphotransferases (figure 8). Finally, SMS1 and SMS2 adopt a membrane topology whereby the putative active site residues are facing the exoplasmic leaflet (figure 13), hence the side of the membrane where SM synthesis was found to occur.

A multiplicity of SM synthase genes appears widely spread among organisms generating SM. Human SMS1 and SMS2 are highly conserved in mammals and evidence was found for the existence of up to three functional homologues in the nematode *C. elegans.* Moreover, two orthologous sequences in the human malaria parasite *P. falciparum* were identified according to the process of the present invention, suggesting that this lower eukaryote too contains different SM synthases encoded by separate genes. SM synthesis in *P. falciparum* occurs in the Golgi apparatus as well as in the network of tubovesicular membranes (TVM) emerging from the parasitophorous vacuolar membrane during intraerythrocytic development (Elmendorf and Haldar, 1994). A differential sensitivity to 1-phenyl-2-acylamino-3-morpholino-1-propanol has been taken as evidence that the Golgi- and TVM-associated activities correspond to different enzymes (Kauer et al., 1995). Our current findings provide a novel opportunity to test this prediction and may facilitate the development of new drugs against parasites as for instance malaria.

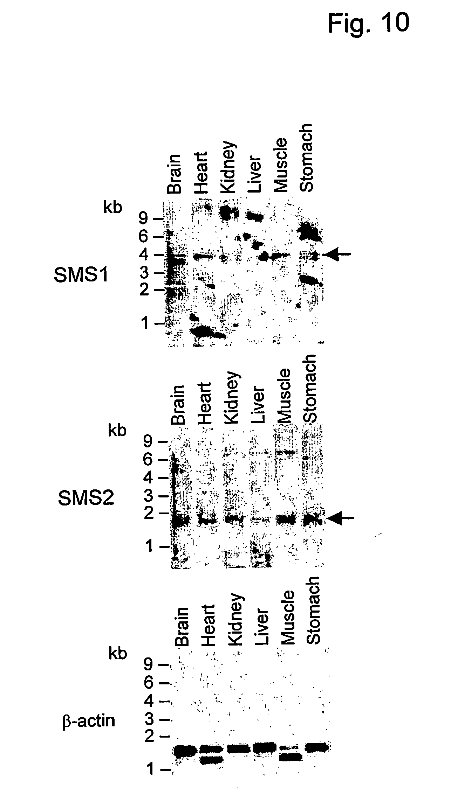

The uniform tissue distributions of transcripts for SMS1 and SMS2 in humans (figure 10) suggest that most mammalian cell types would contain both SM synthase isoforms. Biochemical analysis of human SMS1 and SMS2 thus far revealed no fundamental differences in enzymatic properties, even though the possibility that substrate preferences are lost when enzymes are assayed in a heterologous, detergent-containing system cannot be excluded. For now, the most striking difference between SMS1 and SMS2 concerns their subcellular localisation. While SMS1 seems to represent the well-known Golgi-associated SM synthase, SMS2 primarily resides at the plasma membrane. Hence, SMS1 would be proximal to SMS2 with respect to receiving newly synthesized ceramide from the ER. A challenging prospect is that, whereas the Golgi enzyme would be responsible for generating the bulk of cellular SM, the second enzyme may serve a principal role in signal transduction at the plasma membrane. The activation of a sphingomyelinase (SMase) and subsequent liberation of ceramide at the plasma membrane has been recognised as an important signalling event in the regulation of fundamental cellular processes that include cell cycle arrest, differentiation and apoptosis (Kolesnic and Hannun, 1999; Pettus et al., 2002; Andrieu-Abadie and Levade, 2002). By converting ceramide back to SM, plasma membrane-associated SM synthase may attenuate SMase-induced signalling. This reaction would produce as a side product DAG, which is a signalling molecule in its own right (Brose and Rosenmund, 2002).
Hence, the presence of a SM synthase at the plasma membrane complicates a proper understanding of the role of ceramide as a specific signal-transducer.

A current bottleneck for elucidating the role of SM hydrolysis in cellular signalling is the unambiguous identification of the agonist-stimulated SMase. Both neutral and acid SMases have been implicated, but their precise cellular roles in SM hydrolysis and signalling remain to be clarified (Andrieu-Abadie and Levade, 2002). Since the plasma membrane-associated SM synthase is capable of catalysing the reverse reaction of SM synthesis (van Helvoort et al., 1994), it is tempting to speculate that it may execute part of the signalling events currently attributed to ligand-induced SMase. The observation that both plasma membrane- and Golgi-associated SM synthases exhibit reverse activity brings up the question of how the directionality of these enzymes is regulated. One possibility is that the direction of the reaction is primarily determined by the relative concentrations of ceramide and DAG in the membrane. On the other hand, given the biological importance attributed to DAG and ceramide, one may expect the interconversion of these molecules to be subjected to a more elaborate form of control. Indeed, it has been shown that activation of SM synthesis in primary astrocytes is an early event associated with the mitogenic activity of basic fibroblast growth factor (Riboni et al., 2001) and that the onset of TNFα-induced apoptosis in rhabdomyosarcoma cells is preceded by TNF-dependent inhibition of SM synthesis (Bourteele et al., 1998). Mammalian SMS1 contains a predicted SAM (sterile alpha motif) domain at the amino-terminus (residues 7-63 in human SMS1) that might provide a means for the enzyme to interact with regulatory proteins (Schultz et al., 1997). The present identification of a family of animal SM synthases offers unprecedented opportunities to further clarify the biological significance of SM metabolism and its regulation.

SM synthases contain four highly conserved sequence motifs (figure 8). Two of these, D3 and D4, are similar to the C2 and C3 phosphatase domains in LPPs and include the putative catalytic histidine and aspartate residues implicated in LPP-mediated hydrolysis of lipid phosphate esters (Neuwald, 1997) This suggests a working mechanism for SM synthases in which the catalytic triad previously described for LPPs would function in a similar fashion. The reaction would proceed via the following steps: (1) binding of a two-chain choline-phospholipid (PC or SM) to a unique binding site, (2) nucleophilic attack on the lipid-phosphate ester bond by the histidine in D4 assisted by the conserved aspartate in this motif, (3) formation of a cholinephospho-histidine intermediate and release of DAG or ceramide, facilitated by the histidine in D3 acting as a base, (4) nucleophilic attack of the Cl-hydroxyl of ceramide or DAG on the cholinephospho-histidine intermediate, assisted by the histidine in D3, (5) release of SM or PC from the active site to allow another round of catalysis. This model provides a framework for site-directed mutagenesis and kinetic studies to reveal the working mechanism of SM synthases.

There is a striking parallel between animal SM synthase and IPC synthase in yeast. Like human SMS1, the yeast enzyme is Golgi localised and contains the key D4 motif in the Golgi lumen at the start of the last membrane span (Levine et al., 2000). This suggests that animal SM synthase and yeast IPC synthase may have evolved from a common ancestor. In contrast, the SM synthase recently identified from *Pseudomonas aeruginosa* (Luberto et al, 2003) lacks the key motifs of animal SM synthase and is a soluble, rather than an integral membrane protein. Hence, it appears that SM synthases arose at least twice during evolution.

Database searches using the catalytic site sequences from LPPs yielded, in addition to SM synthases, numerous other proteins containing partially conserved phosphatase motifs. In many cases, the biochemical functions of these proteins are not known.

Given the present findings, the possibility that some of these LPP-like proteins do not serve as phospholipases but instead catalyse novel kinds of synthetic or transphosphatidylation reactions deserves consideration. For example, mammals and insects synthesize ethanolamine phosphorylceramide, a sphingolipid analogous to SM
(Malgat et al., 1986; Rietveld et al., 1999). Whether production of EPC and SM involves a single transferase that accepts both PC and PE as substrates or requires two separate enzymes has remained an open issue. However, *Drosophila* lacks SM and generates only EPC (Rietveld et al., 1999). Our finding that *Drosophila* contains an SMS-related protein, but no SMS proteins, strongly suggests that the SMS-related proteins described in this study function as dedicated EPC synthases. This possibility is currently under further investigation.

Phosphorylcholine (PC), a small haptenic molecule, is found in a wide variety of prokaryotic organisms, i. e. bacteria, and in eukaryotic parasites such as nematodes, as well as in fungi. Linked to parasite-specific glycoprotein glycans or glycolipids, it is assumed to be responsible for a variety of immunological effects, including invasion mechanisms and long-term persistence of parasites within the host. Numerous reports have indicated various effects of PC-substituted molecules derived from parasitic nematodes on signal transduction pathways in B and T lymphocytes, displaying a highly adapted and profound modulation of the immune system by these parasites.

The Nematoda, comprising parasitic and free-living species (e.g. *C. elegans*), can be regarded as promising prototypic systems for structural analyses, immunological studies and biosynthetic investigations. In this context, *Ascaris suum,* the pig parasitic nematode, is an ideal organism for immunological studies and an excellent source for obtaining large amounts of PC-substituted (macro)molecules. *C. elegans,* as a completely genome-sequenced species and expressing parasite analogous PC-substituted structures, together with the possibility for easy *in vitro* cultivation, represents a conceptual model for biosynthetic studies, whereas filarial parasites represent important model systems for human pathogens, especially in developing countries. Current knowledge on the tissue-specific expression of PC epitopes, structural data of glycoprotein glycans and glycosphingolipids bearing this substituent and biological implications for the immune systems of the respective hosts are reviewed by Lochnit et al., 2000. Since the elucidated structures of these compounds are clearly different from those present in the host, the biosynthesis of nematode-specific, PC-substituted compounds would be a promising target for the development of new anthelminthic strategies.

In the present invention it has been shown that modulating the activity of an enzyme of the group of enzymes identified as sphingomyelin synthases, ethanolamine phosphorylceramide synthases, phosphatidylcholine:glycolipid cholinephosphotransferase (further referred to as the group of enzymes) in a cell has effects on cell growth and/or survival. By lowering the activity in cells, the cells respond by a reduction in cell growth, increased sensitivity toward apoptotic stimuli and/or reduced cell survival. Upregulation of the activity induces cell growth, rejuvenation of the cells and/or a reduced sensitivity toward apoptotic signals. An effect is, of course preferably relative to the behaviour of normal cells. These cells typically have effective but not saturating levels of an activity of an enzyme of the group of enzymes and preferably of a sphingomyelin synthase. The possibility of detecting a change in the activity of an enzyme of the group, that can be correlated to a particular amino acid sequence has led the inventors to methods for determining whether a compound is capable of modulating an enzymatic activity displayed by a cell, said activity comprising an activity of an enzyme of the group of enzymes identified as sphingomyelin synthases, ethanolamine phosphorylceramide synthases, phosphatidylcholine:glycoprotein cholinephosphotransferase and phosphatidylcholine:glycolipid cholinephosphotransferase, said method comprising providing said cell with a nucleic acid encoding a polypeptide comprising the amino acid motifs (a), (b) and (c), contacting said cell with said compound and determining whether said enzymatic activity is modulated. In a preferred embodiment said method comprises detecting whether said activity is inhibited. Thus the invention further provides an inhibitor of a enzyme activity of the group, preferably a sphingomyelin synthase of the invention for use as a cell death promoter. Preferably said cell is a parasitic cell or a mammalian tumor cell, preferably a human tumor cell. The compound capable of modulating said enzyme activity displayed by the cell, can act on the level of expression of the enzyme, wherein a higher expression results in upregulation of the enzyme activity displayed by the cell and wherein a lower expression results in downregulation of the enzyme activity displayed by the cell.

Alternatively, the compound can act on the activity of the enzyme in the cell, i.e. the compound can increase the activity of the enzyme and thereby upregulate the enzymatic activity displayed by the cell, or the compound can decrease the activity of the enzyme and thereby downregulate the enzymatic activity displayed by the cell.

In a preferred embodiment of a method of the invention, said enzyme activity of the group comprises a sphingomyelin synthase activity. A polypeptide of the invention comprising sphingomyelin synthase activity comprises an amino acid sequence having an amino acid sequence according to formula (a), (b) and (c). In a particularly preferred embodiment a polypeptide of the invention comprising sphingomyelin synthase activity in a mammalian cell (a mammalian sphingomyelin synthase) and having the above mentioned identity to the formulas, further comprises at least 90% sequence identity with ceSMS1 (Seq. ID 14), depicted in figure 9. A polypeptide of the invention comprising sphingomyelin synthase activity in a plasmodium cell (a plasmodium sphingomyelin synthase) and having the above mentioned identity to the formulas, preferably further comprises at least 90% sequence identity with pfSMS1 (Seq. ID 17) or pfSMS2 (Seq. ID 18) depicted in figure 9. In a particularly preferred embodiment a polypeptide of the invention comprising ethanolamin phosphorylceramid synthase activity in a mammalian cell (a mammalian ethanolamin phosphorylceramid synthase) and having the above mentioned identity to the formulas. A polypeptide of the invention comprising ethanolamin phosphorylceramid synthase activity in an insect cell (an insect ethanolamin phosphorylceramid synthase) and having the above mentioned identity to the formulas, preferably further comprises at least 90% sequence identity with dmSMSr (Seq. ID 20) depicted in figure 9. A polypeptide of the invention comprising PC:glycolipid/glycoprotein cholinephosphotransferase activity in a nematode cell (an nematode PC:glycolipid/glycoprotein cholinephosphotransferase) has the above mentioned identity to the formulas.

By providing a cell with a polypeptide comprising the amino acid motifs (a), (b) and (c), having an enzyme activity of the group one produces a cell having more activity of said enzyme than before. This allows for early and easier detection of the effect that compounds have on the activity displayed by the cell. In a preferred embodiment a polypeptide comprising the amino acid motifs (a), (b) and (c), having activity of an enzyme of the group is provided to a cell that did not contain this activity in detectable amounts. Such cells may be generated by inactivating the endogenous genes coding for the polypeptide or cells can be used from species and/or genera that do not contain this activity. Non-limiting examples of such species and/or genera in case of sphingomyelin synthase activity are: drosophila and yeast. Thus in a preferred embodiment the method ulitizes a cell that was deficient in an enzyme activity of the group, which is preferably a sphingomyelin synthase activity, prior to providing the cell with the polypeptide of the invention.
A cell can be any type of cell. A cell can be part of an organism, where one or more of the cells of the organism are provided with (nucleic acid encoding) said polypeptide. The cell can also be a cell of a micro-organism. The micro-organism can be bacterial and is preferably a eukaryotic micro-organism. Yeasts and fungal micro-organisms are preferred.

The compound can be any chemical or biological compound. In one embodiment said compound has an effect on expression of a polypeptide comprising the amino acid motifs (a), (b) and (c), in a cell. Such a compound can be an expression cassette comprises an expressible coding region for said polypeptide. It can also comprise for instance a compound capable of modulating the activity of a promoter for the gene encoding said polypeptide in said cell. In a preferred embodiment said compound is capable of preventing translation of mRNA encoding said polypeptide in said cell. Translation is preferably inhibited through anti-sense RNA or RNAi. In a particularly preferred embodiment translation is inhibited through RNAi. RNAi is a collective term for a response of cells to double stranded RNA. A variety of different types of compounds are able to induce the specific degradation of mRNAs. None of those different types of compounds are herein excluded. In a preferred embodiment the compound comprises siRNA (Elbashir et al., Nature 411, 494-498) or a hairpin RNA. A hairpin RNA can be introduced directly into the cell or be expressed by a gene encoding the hairpin.

In another aspect the invention provides the use of a nucleic acid encoding a polypeptide according to the invention, as a probe. In a preferred aspect the invention provides the use of an oligonucleotide specific for a nucleic acid sequence encoding a polypeptide according to the invention, for detecting said sequence.

Detection of the sequence can be for any purpose and is preferably for the purpose of assessing whether a cell comprises an enzyme activity of the group of enzymes mentioned above, preferably sphingomyelin synthase activity.

In another aspect the invention provides a nucleic acid encoding a polypeptide comprising the amino acid motifs (a), (b) and (c), for use in enhancing cell survival and/or cell growth. It has been found that increasing the enzymatic activity displayed by a cell of an enzyme of the group of enzymes, has the effect that the secretion pathway of the cell is stimulated. This in yet another aspect the invention provides a method for at least in part improving the yield of an secretion product of a cell comprising providing said cell with a polypeptide comprising the amino acid motifs (a), (b) and (c), or a nucleic acid such a polypeptide. In a preferred embodiment said polypeptide comprises sphingomylin synthase activity. In this embodiment said cell is preferably a mammalian cell or a cell of a eukaryotic micro-organism.

A method for determining whether a compound is capable of modulating an enzymatic activity displayed by a cell, said activity comprising an activity of an enzyme of the group of enzymes identified as sphingomyelin synthases, ethanolamine phosphorylceramide synthases, phosphatidylcholine:glycoprotein cholinephosphotransferase and phosphatidylcholine: glycolipid cholinephosphotransferase can be performed using any method for the detection of said enzymatic activity. In a preferred embodiment the method for detecting said enzyme activity comprises providing said cell or a fraction thereof with a labelled substrate for said enzyme. Said substrate is preferably a substrate for sphingomyelin synthase. The method preferably further comprises harvesting sphingolipid from said cell or said fraction and detecting labelled sphingolipid. Detection of said labelled sphingolipid is preferably done using (thin layer) chromatography or mass spectrometry.

Polypeptides comprising the amino acid motifs (a), (b) and (c), are associated with a membrane of the cell. The localization varies with the particular polypeptide. It has been observed that this property can be used to generate chimearic polypeptides having sequences from at least two polypeptides comprising the amino acid motifs (a), (b) and (c), which have an altered distribution in the cell when compared to at least one of the polypeptides said chimearic polypeptide is derived from. Such chimearic polypeptides and nucleic acids encoding the chimearic polypeptides are therefore also included in the invention. Thus the invention provides a method for targeting a first polypeptide comprising the amino acid motifs (a), (b) and (c), to a different cellular compartment comprising providing a cytosolic part of said first polypeptide with a cellular compartment localization signal of a cytosolic part of a second polypeptide comprising the amino acid motifs (a), (b) and (c), wherein said first and said second polypeptide, when unmodified, reside in different cellular compartments. Chimearic polypeptides wherein the at least two original polypeptides reside in essentially the same cellular compartment are also included in the invention. It is also possible to attach known cellular localization signals to a polypeptide of the invention. Such known cellular localization signals, for instance, an endoplasmatic reticulum retention signal do not have to be derived from a polypeptide of the invention. The cellular localization signal is preferably present in the cytosolic C-terminal end of a polypeptide of the invention. Thus preferably the cytosolic C-terminal end of said first polypeptide is provided with a cellular compartment localization signal of a cytosolic part of the second polypeptide. Preferably said cytosolic part of said second polypeptide comprises the C-terminal end of a polypeptide comprising the amino acid motifs (a), (b) and (c). Another way to target a polypeptide of the invention to a different compartment is to remove the cellular localization signal from the cytosolic part (preferably the C-terminal cytosolic part) of the polypeptide.

The polypeptide can be targeted to any cellular compartment having a membrane and be active there. In a preferred embodiment said cellular compartment comprises a plasma membrane, an endosomal compartment, a Golgi, an endoplasmatic reticulum or a combination thereof. Cellular localization signals do not have be absolute in that there can be cases wherein detectable amounts of polypeptide are spread over at least two cellular compartments. A polypeptide is said to be targeted to a different compartment when, compared to the original cellular localization of one of the polypeptides of the invention, the chimearic polypeptide is detectable in at least on other compartment or is not detectable in a compartment where one of the single polypeptides is detectable. A special case of targeting is where not the localization but rather the relative distribution over compartments changes. For instance, when an percentage X of a polypeptide of the invention is normally present in a first compartment and a percentage Y is present in a second compartment. In this example targeting is said to have taken place when the percentage in at least one of said at least two compartments is altered by more than 30%. Preferably, the change is reciprocal.

Considering that the cellular compartment localization signal of a polypeptide of the invention is predominantly determined by a C-terminal cytosolic part of said polypeptide it is preferred that said cellular compartment localization signal of the C-terminal cytosolic part of said second polypeptide replace the C-terminal cytosolic part of said first polypeptide.

It will be clear to the skilled in the art that by the present invention yielding purified cholinephosphotransferases and ethanolaminephosphotransferases a new field of development for new compounds with valuable properties as for instance drugs has been made available. The present invention will further be elucidated by the following examples without in any way restricting the broad scope thereof.

### Materials and methods

### Chemicals

Sphingosyl-(NBD-hexanoyl)-phosphocholine (NBD-SM) and (NBD-hexanoyl)-ceramide (NBD-Cer) were from Molecular Probes (Eugene, OR) and Oleoyl-(NBD-hexanoyl)-phosphocholine (NBD-PC) from Avanti Polar Lipids (Alabaster, AL). [methyl-¹⁴C]-choline was from ICN Biomedicals (Irvine, CA) and L-3-phosphatidyl [N-methyl-³H]-choline,1,2-dipalmitoyl from Amersham Pharmacia (Piscataway, NJ). NBD-DAG was produced from NBD-PC by treatment with phospholipase C from B. cereus as described (Trotter, 2000). Egg L-α-phosphatidylcholine, lyso-phosphatidylcholine, L-α-phosphatidylethanolamine, L-α-phosphatidylglycerol, L-α-phosphatidylserine, soybean L-α-phosphatidylinositol, dioctanoyl L-α-phosphatidic acid, SM (from bovine brain), choline, phosphocholine, cytidine 5'-diphosphocholine, O-tricyclo[5.2.1.0^{2,6}]dec-9-yl dithiocarbonate potassium salt (D609), N-ethylmaleimide (NEM) and phospholipase C (Bacillus cereus) were from Sigma Aldrich (St. Louis, MO).

### Selection, cloning and expression of CSS sequences

Animal entries in the Swiss-Prot/TrEMBL protein sequence database were searched using Prosite (http://www.expasy.org/tools/scanprosite) against sequence motif H-[YFWH]-X₂-D-[VLI]-X₂-[GA]-X₃-[GSTA] and then further selected based on three additional criteria as described under Results. Candidate SM synthases (CSS) were assembled into different groups by multiple sequence alignments and phylogenetic trees generated with ClustalW and PHYLIP. Open reading frames (ORFs) in selected CSS sequences were PCR amplified using Taq polymerase (MBI Fermentas, Hanover, MD) according to information provided in Table I. PCR products were cloned into yeast expression vector pYES2.1/V5-His-TOPO (Invitrogen Corporation, Carlsbad, CA) and the resulting plasmids used to transform S. cerevisiae strain IAY11 (MATα ura3-52 his3Δ200 leu2-3, -112 trpΔ901 ade2-101 ade3-Δ853, provided by Ian Adams, MRC-LMB, Cambridge, UK). Transformants were grown in complete minimal uracil dropout medium containing 2% (w/v) galactose. Expression of CSS proteins was verified by Western blot analysis with mouse anti-V5 antibodies (Invitrogen). PCR amplified ORFs of human SMS1 and SMS2 were cloned into mammalian expression vector pcDNA3.1/V5-His-TOPO (Invitrogen) and the resulting plasmids (SMS1-V5/pcDNA3.1 and SMS2-V5/pcDNA3.1) used to transfect HeLa cells.

### SM synthase assay on cell lysates

CSS-expressing yeast cells were lysed at 100 OD₆₀₀/ml by bead bashing at 4° C in RB1 buffer (120 mM K-glutamate, 15 mM KCl, 5 mM NaCl, 2 mM MnCl₂, 2 mM MgCl₂, 20 mM Hepes-KOH, pH 7.2) containing freshly added protease inhibitors (Holthuis et al., (1998).

Lysates were centrifuged at 700 g for 10 min at 4 ° C and supernatants (0.25 ml per reaction) preincubated with NEM (1 mM) for 10 min at 37 ° C. The reaction was initiated by addition of 5 nmol of NBD-Cer in 0.1 ml RB1 buffer and incubation continued for 15 min. Lipids were extracted by addition of 1.7 ml chloroform:methanol (1:2.2), dried under N₂ and subjected to butanol/water partitioning. Lipids recovered from the butanol phase were separated by one-dimensional TLC for 30 min in solvent I: chloroform/methanol/4.2 M NH₄OH (9:7:3, v/v), followed by 30 min in solvent II: chloroform/methanol/acetic acid (45:30:7, v/v). Fluorescent images were recorded on a STORM 860 Imaging Analysis System (Molecular Dynamics, Sunnyvale, CA) and analysed with ImageQuant software.

### Metabolic labelling

CSS-expressing yeast cells (0.5 OD₆₀₀) were inoculated in 5 ml medium containing 10 µCi [*methyl*-¹⁴C]-choline and 10 nmol NBD-Cer. Cells were grown for 16 h at 30 ° C, washed in water, and lipids were extracted by bead bashing in H₂O/methanol/chloroform (5:16:16, v/v). The organic extracts were dried under N₂, subjected to butanol/water partitioning, and lipids recovered from the butanol phase were deacylated by mild base treatment using 0.2 N NaOH in methanol (60 min, 30 ° C). After neutralizing with 1 M acetic acid, lipids were extracted with chloroform and separated by two-dimensional TLC using solvent I in the first, and solvent II in the second dimension. Radio-labelled lipids were detected by exposure to BAS-MS imaging screens (Fuji Photo Film Co., Japan) and read out on a BIO-RAD Personal Molecular Imager.

### Preparation of detergent extracts

SMS1/SMS2-expressing yeast cells were lysed at 100 OD₆₀₀/ml by bead bashing at 4°C in lysis buffer (50 mM Tris-HCl, pH 7.0, 1 mM EDTA, 0.3 M sucrose) containing protease inhibitors. Post nuclear supernatants were prepared as above and loaded onto a 60% (w/w) sucrose cushion and then centrifuged at 100.000 g for 60 min at 4 ° C. Membranes derived from ± 500 OD₆₀₀ of cells were resuspended in 1 ml ice-cold extraction buffer (50 mM Tris-HCl, pH 7.0, 10% glycerol, 1% Triton X-100, 1 mM MnCl₂) containing protease inhibitors, incubated for 60 min at 4 ° C while rotating and then centrifuged at 100.000 g for 60 min at 4° C. Detergent extracts were diluted to a final protein concentration of 1.5 mg/ml in extraction buffer and 100 µl aliquots were snap frozen in liquid N₂ and stored at -80 ° C.

### SM synthase and reverse transferase assays on detergent extracts

Detergent extracts were diluted 5- to 8-fold in extraction buffer, and 50 µl mixed with 5 µl of a 1.2 mM head group donor stock (e.g. CDP-choline, PC, PI) or with 15 µCi (350-700 fmol) of [³H]-choline-containing PC prepared in extraction buffer. Following a 15 min preincubation at 37 ° C, reactions were started by addition of 220 µl RB2 buffer (50 mM Tris-HCl, pH 7.0, 5 mM MgCl₂, 1 mM MnCl₂, 1 mM NEM) containing 5 nmol NBD-ceramide (SM synthase assay) or 5 nmol NBD-DAG (reverse transferase assay) and incubated for 60 min at 37 ° C. Lipids were extracted, separated by TLC, and analysed as above.

### Mass spectrometry

Detergent extracts (400 µl) were mixed with bovine brain ceramide (180 µM) and egg PC (500 µM in a total volume of 500 µl. After 15 min at 37 °C, reactions were diluted 4-fold in RB2 buffer and then incubated for another 60 min at 37 °C. Lipids were extracted in chloroform:methanol (1:2.2) and subjected to butanol/water partitioning. Residual Triton X-100 was removed by solid phase extraction on Si-60 columns (500 mg stationary phase) in acetone. Phospholipids were eluted in methanol/chloroform (19:1, v/v), dried under N₂, dissolved in methanol/chloroform (2:1, v/v) and then separated by HPLC on two 250 x 4.6 mm Lichrosphere RP-18 end-capped columns (Merck, Darmstadt, Germany) in series (Brouwers et al., 1998). The column effluent was split in a 10:1 ratio, with the smaller fraction going to the mass spectrometer. Mass spectrometry was performed on a Sciex API-365 triple quadrupole mass spectrometer (Applied Biosystems, Nieuwerkerk a/d IJssel, The Netherlands). Positive ions were generated by a turbo-ion spray ionisation source operating at +5.5 kV ionisation potential. N₂ (2 l/min) was used as drying gas at a temperature of 350 °C. The declustering potential (cone voltage) was set to 45V and the focus potential to 220V. Mass spectra from mass to charge ratio (m/z) 650 amu to 950 amu were recorded at a speed of 120 amu/s.

### Mammalian cell transfection and immunofluorescence microscopy

HeLa cells were cultured in DMEM medium containing 10% fetal calf serum. Cells were grown on glass coverslips to 40% confluence and then transfected with human SMS1-V5/pcDNA3.1, SMS2-V5/pcDNA3.1 and *myc-*tagged sialyltransferase/pCB7 constructs using Lipofectamine 2000 (Invitrogen). After 48 h, cells were fixed in 3% paraformaldehyde/PBS and processed for immunofluoresence microscopy (see Supplementary materials for details). For cell surface biotinylation, cells were incubated twice with 0.5 mg/ml sulfo-NHS-SS-biotin (Pierce) in PBS for 20 min on ice, quenched in 10 mM glycine/PBS for 20 min and then washed in PBS prior to fixation. Immunostaining was with rabbit anti-V5 antibodies (Sigma), mouse anti-V5 antibodies (Invitrogen), mouse 9E10 anti-myc antibody (SanverTech, CA), rabbit anti-biotin antibodies (Rockland, Gilbertsville, PA), FITC- or Texas Red-conjugated goat anti-rabbit and goat anti-mouse antibodies (Jackson Laboratories, West Grove, PA). Images were obtained using a Nikon D-εclipse C1 confocal miscroscope.

### Protease protection assay

HeLa cells transfected with human SMS1-V5 or SMS2-V5 constructs were washed, scraped and then lysed in ice-cold PNS buffer (120 mM K-glutamate, 15 mM KCl, 5 mM NaCl, 2 mM MnCl₂, 0.8 mM CaCl₂, 2 mM MgCl₂, 1.6 mM EDTA and 20 mM Hepes-KOH, pH 7.2) by 20 passages trough a 26³/₄ gauge needle. The homogenate was treated with 8 mM trypsin (Sigma Aldrich) in the presence or absence of 0.4% Triton X-100 for 30 min at 37 °C. Alternatively, cells were only washed and then trypsin-treated in PNS buffer as above. Homogenates and cell suspensions were transferred on ice, trypsin inhibitor (Sigma Aldrich) was added to 160 mM and samples were processed for Western blot analysis using mouse anti-V5 antibodies and rabbit anti-p24 protein (Gommel et al., 1999, provided by B. J. Helms, Faculty of Veterinary Medicine, Utrecht).

### SDS-PAGE and Western Blot Analysis

Protein extracts were prepared in SDS/Urea sample buffer (20 mM Tris-HCl, pH 6.8, 1 mM EDTA, 4.5 M urea, 2.5% SDS, 0.01% bromophenol blue) containing fresh protease inhibitors (Holthuis et al., (1998). Extracts were heated for 10 min at 50 ° C, centrifuged briefly at 14,000 g and then resolved by SDS-PAGE on 10% polyacrylamide mini-gels. For Western blotting, nitrocellulose transfers were blocked for 90 min in PBS, 5% Protifar (Nutricia, Zoetermeer, The Netherlands), 0.2% Tween-20 (Blotto). Primary antibody incubations were performed for 1 h in Blotto. Detection was with horsheradish peroxidase-conjugated goat anti-rabbit or anti-mouse IgG, using enhanced chemiluminescence (Amersham Pharmacia Biotech).

### Immunofluorescence Microscopy

HeLa cells were grown on coverslips, transfected with human SMS1-V5/pcDNA3.1, SMS2-V5/pcDNA3.1 and myc-tagged sialyltransferase/pCB7 (Sprong et al., 2001). 48 h after transfection, cells were fixed in 3% paraformaldehyde/PBS for 30 min at room temperature, quenched in PBS containing 50 mM NH₄Cl, and then blocked and permeabilized for 1 h in PBS containing 0.5% BSA, 0.1% saponine (blocking buffer). Cells were labelled with mixtures of primary antibodies (1:200) for 1 h in blocking buffer, washed for 45 min in blocking buffer with three buffer changes, and then incubated for 20 min with 10% goat serum in blocking buffer. For immunostaining of intact cells, incubation with primary antibodies occurred before fixation and was performed for 1 h at 4°C in PBS containing 0.5% BSA. Cells were counterstained with fluorescently labelled secondary goat antibodies (1:75) for 30 min in blocking buffer, washed in blocking buffer for 45 min with three buffer changes, and then rinsed briefly in PBS and water. Mounting was in Mowiol 4-88 (Calbiochem, La Jolla, CA) containing 2.5% 1,4-diazabicyclo[2.2.2.]octane (Sigma).

### Example 1

### Identification of candidate SM synthases from the animal database

Figure 1A shows an outline of the bioinformatics approach used to identify candidate sequences for SM synthases from the animal database. Candidate SM synthases were identified based on the following criteria: 1) presence of a sequence motif, H-[YFWH]-X₂-D-[VLI]-X₂-[GA]-X₃-[GSTA], shared by previously characterised LPPs and Aurlp homologues; 2) biochemical function should be unknown; 3) no structural homologues in S. cerevisiae, since this organism lacks SM; 4) presence of multiple (>2) transmembrane domains, since the enzyme mechanism is intramembranous and because LPPs and Aurlp proteins have 6 predicted membrane spans. Sequences conforming to all 4 criteria were subsequently used as queries in a BLAST search to track down homologous sequences that were missed in the initial search due to deviations in the LPP/Aur1p motif. This approach yielded 9 human, 9 mouse and 9 *C. elegans* sequences that could be grouped into three major protein families, designated CSS1, CSS2 and CSS3 (for candidate SM synthase family 1-3; Figure 1B, C). Except for the presence of a common sequence motif, CSS proteins displayed no significant sequence similarity to Aur1p proteins or to LPP family members with known biochemical functions. However, human CSS1β1 is identical to PRG1, a neuron-specific candidate phosphatidic acid phosphatase with a role in axon growth and regenerative sprouting (Brauer et al., 2003)
Database accession numbers of CSS proteins are listed in the legend of Figure 1.

### Example 2

### A subset of CSS3 family members displays SM synthase activity

To investigate whether any of the three CSS families indeed contained SM synthases, the open reading frames of human, mouse and *C. elegans* members for which full-length cDNAs could be obtained were cloned into a yeast multicopy, GAL1 promotor plasmid in frame with a carboxy-terminal V5 epitope. The resulting plasmids were used to transform wild-type yeast and the transformants were shifted to galactose-containing medium to induce expression of recombinant proteins. Expression of proteins was verified by Western blot analysis using anti-V5 antibodies (Figure 2A). Thus, 13 of the 27 selected CSS sequences were expressed and analysed for SM synthase activity (marked by asterisks, Figure 1C). To this end, yeast cells expressing CSS protein were lysed and incubated with fluorescent C₆-NBD-ceramide (NBD-Cer), a known substrate of mammalian SM synthase. Incubations were performed in the presence of N-ethylmaleimide (NEM), a potent inhibitor of yeast-associated SMases that does not affect mammalian SM synthase (Figure 3). Reaction mixtures were next subjected to one-phase lipid extraction and the lipids separated by one-dimensional TLC.

Figure 2B shows that in lysates of control cells NBD-Cer was converted exclusively into NBD-IPC. The same was true for lysates of cells expressing members of the CSS1 and CSS2 protein families. However, in lysates of cells expressing human CSS3α1, human CSS3α2, *C. elegans* CSS3α1 or *C. elegans* CSS3α2, NBD-Cer was converted to a second product with an Rf value identical to that of NBD-SM. The identity of this product as NBD-SM was confirmed by electrospray ionisation tandem mass spectrometry (ESI-MS/MS; data not shown). Moreover, metabolic labelling of cells expressing human CSS3α1 or CSS3α2 with [¹⁴C]-choline in the presence of NBD-Cer resulted in the production of radio-labelled NBD-SM (Figure 2C). When NBD-Cer was omitted, we noticed that CSS3α2-expressing cells generated small amounts of [¹⁴C]-choline-labelled lipids that were absent in control cells and distinct from PC and lysoPC. In addition, these lipids were resistant to mild alkaline hydrolysis, suggesting that they represent phytoceramide-based SMs (phytoSM; Figure 2D). Importantly, ESI-MS revealed that bovine brain ceramides incubated with detergent extracts of CSS3α2-expressing cells, but not control cells, are converted to SM (Figure 4). Together, these results indicate that CSS3α proteins do not only use NBD-Cer, but also recognise naturally occurring ceramides as substrates for SM synthesis.

Human CSS3α1 and CSS3α2 share 57% sequence identity and are highly conserved in mammals (human - mouse > 90%). Based on the results presented herein, we propose to rename these proteins SMS1 and SMS2, respectively. *C. elegans* CSS3α1 (ceSMS1) and CSS3α2 (ceSMS2) share 22-27% sequence identity with human SMS1 and SMS2, and apparently represent their functional counterparts in the nematode (Figure 2B). *C. elegans* contains a third, CSS3α-related protein, termed ceCSS3α3. Although we have not tested this protein for SM synthase activity, the finding implies that *C. elegans* is equipped with three independent SM synthases (Figure 1C). BLAST searches for orthologous sequences identified two proteins in the human malaria parasite Plasmodium falciparum (PlasmoDB accession no. MAL6P1.177 and MAL6P1.178; see also below). Hence, a multiplicity of SM synthase genes appears a general feature of organisms generating SM.

Apart from CSS3α/SMS proteins, the CSS3 family contains a second cluster of CSS3β or SMS-related (SMSr) proteins with members in *humans,* mice, *C. elegans, and Drosophila* (Figure 1C). *Human* SMSr is highly conserved in mammals (human-mouse: 95%), shares > 40% sequence identity with its orthologues in *C. elegans* and *Drosophila,* and is about 34% identical to human SMS1 and SMS2. Heterologous expression of human or *C. elegans* SMSr did not yield any detectable SM synthase activity (Figure 2B, right panel). The *C. elegans* CSS3γ protein, renamed SMSdr, is only distantly related to SMS and SMSr proteins (<22% identical; see also Figure 1C) and its ability to mediate SM synthesis was not tested.

### Example 3

### Human SMS1 and SMS2 function as PC:ceramide cholinephosphotransferases with reverse activity

SM synthesis in animals proceeds by the liberation of phosphorylcholine from PC and its subsequent transfer onto the primary hydroxyl of ceramide. To establish whether human SMS1 and SMS2 function as PC:ceramide cholinephosphotransferases, their enzymatic characteristics were analysed in more detail. Hence, different donors of choline-P were tested as substrates for SM synthesis. To this end, detergent extracts of cells expressing human SMS1 or SMS2 were incubated with NBD-Cer and the formation of NBD-SM was monitored by TLC. A dilution of extracts proved necessary to render SMS-mediated synthesis of NBD-SM dependent on externally added head group donors (Figure 5). Under these conditions, free phosphorylcholine (Ch-P) and CDP-choline (CDP-Ch) did not support SMS1- or SMS2-mediated SM synthesis (Figure 6A). PC, on the other hand, was efficiently recognised as substrate. SM itself was also used as a donor of the phosphorylcholine group. Lyso-PC was a very poor substrate. These results suggest that human SMS1 and SMS2 are transferases that require two fatty chains on the choline-P donor molecule in order to be recognised efficiently as a substrate.

### Example 4

### The reaction of human SMS1 or SMS2 with non-choline phospholipids

We next investigated the possibility that human SMS1 or SMS2 would use non-choline phospholipids as substrates. None of the phospholipids tested (PE, PI, PS, PA, PG) other than PC supported SM formation (Figure 6A). Moreover, when extracts of cells expressing SMS2 were incubated with PC containing [³H]-choline, the formation of radiolabelled NBD-SM was observed (Figure 6B). Hence, it appears that SMS1 and SMS2 directly and specifically recognise the choline head group on their substrates.

### Example 5

### The reaction of sphingomyelin and diacyl glycerol

Previous work suggested that mammalian SM synthase is also capable of catalysing the reverse reaction, namely the formation of PC from SM and DAG (Marggraf and Kanfer, 1984; van Helvoort et al., 1994).

To investigate whether this was also the case for human SMS1 and SMS2, extracts of cells expressing these proteins were incubated with NBD-DAG and the formation of NBD-PC monitored by TLC. Addition of SM induced NBD-PC formation in extracts of SMS1- or SMS2-expressing cells, but not in control cell extracts (Figure 7). Strikingly, PC itself proved more efficient than SM in stimulating SMS1- or SMS2-dependent NBD-PC formation. In contrast, addition of non-choline phospholipids (e.g. PI) had no effect. These data suggest that human SMS1 and SMS2, rather than functioning strictly as SM synthases, are transferases capable of using PC and SM as phosphocholine donors to produce PC or SM, dependent on the relative concentrations of DAG and ceramide as phosphocholine acceptors, respectively.

Mammalian SM synthase was found to be sensitive to the bacterial PC-phospholipase C inhibitor, D609 (Luberto and Hannun, 1998).
In lysates of SMS1- or SMS2-expressing yeast cells, D609 inhibited SM synthesis in a dose-dependent manner. The extent of inhibition observed for SMS1-mediated SM synthesis (50% at 100 g/ml D609) was comparable to that reported for mammalian SM synthase (Luberto and Hannun, 1998). SMS2 proved two-fold less sensitive to the drug.

Hence, human SMS1, SMS2 and the mammalian SM synthase activity described in the literature share many enzymatic characteristics.

### Example 6

### Structure and expression of human SMS1 and SMS2

Figure 8A shows a sequence alignment of human SMS1, SMS2 and SMSr. Hydrophobicity analysis predicted six membrane-spanning alpha helices connected by hydrophilic regions that would form extramembrane loops. A comparative analysis with SMS and SMSr sequences from mice, *C. elegans, P. falciparum* and *Drosophila* revealed that the number and spacing properties of transmembrane helices are well conserved. In addition, SMS proteins contain four highly conserved sequence motifs, designated D1, D2, D3 and D4 (Figure 8A and B, residues highlighted in blue). Motifs D3 (C-G-D-X₃-S-G-H-T) and D4 (H-Y-T-X-D-V-X₃-Y-X₆-F-X₂-Y-H) are similar to the C2 and C3 motifs in LPPs (shared residues highlighted in red) and include the histidine and aspartate residues (underlined) that form a catalytic triad mediating the nucleophilic attack on the lipid phosphate ester bond Neuwald (1997).

As in LPPs, these residues are juxtaposed to transmembrane segments 4 and 6 of the SMS proteins and consequently would be oriented toward the same side of the membrane. This would suggest that motifs D3 and D4 are part of the catalytic site responsible for liberating cholinephosphate from PC during SM synthesis. Motifs D1 (P-L-P-D) and D2 (R-R-X₈-Y-X₂-R-X₆-T), on the other hand, appear entirely unique to SMS proteins and are located in the first extramembrane loop and third transmembrane helix, respectively (Figure 8A). The SMSr proteins found in humans, mouse, Drosophila and *C. elegans* each contain exact copies of the D1 and D3 motifs, yet exhibit one or more conserved amino acid substitutions in motifs D2 and D4 (Figure 8A and B, residues highlighted in white on grey.

Previous work showed that some members of the mammalian LPP superfamily are expressed in only a limited set of tissues (Waggoner et al., 1999, Brauer et al, 1997).
Since differences in tissue distribution would provide a possible explanation for the existence of two different SM synthase isoforms in mammals, we investigated the expression profiles of human SMS1 and SMS2. As shown in Figure 10, Northern blot analysis detected the presence of a low abundant 3.8 kb transcript for SMS1 in human brain, heart, kidney, liver, muscle and stomach. A major 1.9 kb transcript for SMS2 was expressed to a similar level in all of the above human tissues. These results suggest that human SMS1 and SMS2 are encoded by ubiquitously expressed genes.

### Example 7

### Subcellular localisation and membrane topology

SM synthesis occurs in the Golgi complex as well as at the plasma membrane of mammalian cells. Endosomes have been put forward as another major site of SM synthesis (Kallen et al., 1994) but this has been disputed (van Helvoort et al., 1994).

It is not known whether SM synthase activity detected at these locations is due to the presence of more than one iso-enzyme in the cell. This led us to examine the subcellular distribution of V5-tagged versions of human SMS1 and SMS2 in transfected HeLa cells using immunofluorescence microscopy. As shown in Figure 11A, SMS1 was concentrated in the perinuclear region where it displayed extensive colocalisation with sialyltransferase, a marker of trans Golgi cisternae. This colocalisation was also observed in cells treated with nocodazole, a drug causing fragmentation of the Golgi by disrupting the microtubular network, hence confirming the association of SMS1 with the Golgi apparatus. SMS2 displayed a different localisation pattern and was primarily concentrated at the plasma membrane (Figure 11B). A portion of SMS2 was also found in the perinuclear region where it colocalised with sialyltransferase (see Figure 12). This Golgi-associated pool of SMS2 unlikely represents newly synthesized material en route to the cell surface, since it was also observed in cells after a 4-hour chase with cycloheximide. There was no substantial colocalisation of SMS2 with markers of the endosomal/lysosomal system (e.g. EEA1, CD63, internalised transferrin; data not shown). Whether SMS2 cycles between the Golgi and the plasma membrane, and thereby passes through endosomes remains to be established.

Since SM synthesis takes place in the exoplasmic leaflet of the Golgi and the plasma membrane (Futerman et al., 1990; van Helvoort et al., 1994), the putative catalytic residues in motifs D3 and D4 of SMS1 and SMS2 would be oriented toward the Golgi lumen and cell surface, respectively, whereas their termini would be located on the opposite, cytosolic side of the membrane (Figure 13A). To test this prediction, we investigated the sidedness of the V5-tagged COOH termini of human SMS1 and SMS2 by protease protection analysis. When HeLa cells transfected with the SMS2-V5associated type I membrane protein, p24 construct were trypsinised, the V5 tag remained intact, unless cells were lysed or treated with detergent prior to incubation with the protease (Figure 13B). Trypsinisation of lysates from SMS1-V5 expressing cells in the absence of detergent resulted in a complete removal of the V5 tag. Under these conditions, the Golgi-associated type I membrane protein, p24 (Gommel et al., 1999)
was largely protected. Collectively, these results indicate that the COOH termini of SMS1 and SMS2 are cytosolic. Consequently, the putative active site residues in these proteins would be positioned on the exoplasmic leaflet (Figure 13A), hence where SM synthesis was found to occur. Together, these findings demonstrate yet another level of similarity between SMS1, SMS2 and the SM synthase activity previously described in mammalian cells.

### References

Andrieu-Abadie, N. and Levade, T. (2002) Sphingomyelin hydrolysis during apoptosis. Biochim. Biophys. Acta, 1585, 126-134.
Bankaitis, V.A. (2002) Cell biology. Slick recruitment to the Golgi. Science, 295, 290-291.
Bourteele, S., Hausser, A., Doppler, H., Horn-Muller, J., Ropke, C., Schwarzmann, G., Pfizenmaier, K. and Muller, G. (1998) Tumor necrosis factor induces ceramide oscillations and negatively controls sphingolipid synthases by caspases in apoptotic Kym-1 cells. J. Biol. Chem., 273, 31245-31251.
Brauer, A.U., Savaskan, N.E., Kuhn, H., Prehn, S., Ninnemann, O. and Nitsch, R. (2003) A new phospholipid phosphatase, PRG-1, is involved in axon growth and regenerative sprouting. Nat. Neurosci., 6, 572-578.
Brose, N. and Rosenmund, C. (2002) Move over protein kinase C, you've got company. J. Cell Sci., 115, 4399-4411.
Brouwers, J.F., Gadella, B.M., van Golde, L.M. and Tielens, A.G. (1998) Quantitative analysis of phosphatidylcholine molecular species using HPLC and light scattering detection. J. Lipid Res., 39, 344-353.
Dickson, R.C. (1998) Sphingolipid functions in Saccharomyces cerevisiae: comparison to mammals. Annu. Rev. Biochem., 67, 27-48.
Elmendorf, H.G. and Haldar, K. (1994) Plasmodium falciparum exports the Golgi marker sphingomyelin synthase into a tubovesicular network in the cytoplasm of mature erythrocytes. J. Cell Biol., 124, 449-462.
Fukasawa, M., Nishijima, M. and Hanada, K. (1999) Genetic evidence for ATP-dependent ER-to-Golgi apparatus trafficking of ceramide for sphingomyelin synthesis in CHO cells. J. Cell Biol., 144, 673-685.
Futerman, A.H., Stieger, B., Hubbard, A.L. and Pagano, R.E. (1990) Sphingomyelin synthesis in rat liver occurs predominantly at the cis and medial cisternae of the Golgi apparatus. J. Biol. Chem., 265, 8650-8657.
Gommel, D., Orci, L., Emig, E.M., Hannah, M.J., Ravazzola, M., Nickel, W., Helms, J.B., Wieland, F.T. and Sohn, K. (1999) p24 and p23, the major transmembrane proteins of COPI-coated transport vesicles, form hetero-oligomeric complexes and cycle between the organelles of the early secretory pathway. FEBS Lett., 447, 179-185.
Hanada, K., Hara, T., Fukasawa, M., Yamaji, A., Umeda, M. and Nishijima, M. (1998) Mammalian cell mutants resistant to a sphingomyelin-directed cytolysin. J. Biol. Chem., 273, 33787-33794.
Heidler, S.A. and Radding, J.A. (2000) Inositol phosphoryl transferases from human pathogenic fungi. Biochim. Biophys. Acta., 1500, 147-152.
Holthuis J.C., Nichols, B.J., Dhruvakumar, S. And Pelham, H.R. (1998) Two syntaxin homologues in the TGN/endosomal system of yeast. EMBO J., 17, 113 - 126
Holthuis, J.C., Pomorski, T., Raggers, R.J., Sprong, H. and Van Meer, G. (2001) The organizing potential of sphingolipids in intracellular membrane transport. Physiol. Rev., 81, 1689-1723.
Jeckel, D., Karrenbauer, A., Birk, R., Schmidt, R.R. and Wieland, F. (1990) Sphingomyelin is synthesized in the cis Golgi. FEBS Lett., 261, 155-157.
Kallen, K.-J., Allan, D., Whatmore, J. and Quinn, P. (1994) Synthesis of surface sphingomyelin in the plasma membrane recycling pathway of BHK Cells. Biochim. Biophys. Acta, 1191, 52-58.
Kolesnick, R. and Hannun, Y.A. (1999) Ceramide and apoptosis. Trends Biochem. Sci., 24, 224-225.
Lauer, S.A., Ghori, N. and Haldar, K. (1995) Sphingolipid synthesis as a target for chemotherapy against malaria parasites. Proc. Natl. Acad. Sci. USA, 92, 9181-9185.
Levine, T.P., Wiggins, C.A. and Munro, S. (2000) Inositol phosphorylceramide synthase is located in the Golgi apparatus of Saccharomyces cerevisiae. Mol. Biol. Cell, 11, 2267-2281.
Lipsky, N.G. and Pagano, R.E. (1985) Intracellular translocation of fluorescent sphingolipids in cultured fibroblasts. J. Cell Biol., 100, 27-34.
Lochnit, G., Dennis, R. D., and Geyer, R. (2000) Phosphorylcholine substituents in nematodes: structures, occurrence and biological implications. Biol Chem 381, 839-847
Luberto, C. and Hannun, Y.A. (1998) Sphingomyelin synthase, a potential regulator of intracellular levels of ceramide and diacylglycerol during SV40 transformation. J. Biol. Chem., 273, 14550-14559.
Luberto, C., Stonehouse, M.J., Collins, E.A., Marchesini, N., El-Bawab, S., Vasil, A.I., Vasil, M.L. and Hannun, Y.A. (2003) Purification, characterization, and identification of a sphingomyelin synthase from Pseudomonas aeruginosa. J. Biol. Chem., 278, 32733-32743.
Malgat, M., Maurice, A. and Baraud, J. (1986) Sphingomyelin and ceramide-phosphoethanolamine synthesis by microsomes and plasma membranes from rat liver and brain. J. Lipid Res., 27, 251-260.
Malgat, M., Maurice, A., and Baraud, J. (1987) J. Lipid Res. 28, 138-143 Marggraf, W.-D. and Kanfer, J.N. (1984) The phosphorylcholine receptor in the phosphatidylcholine:ceramide cholinephosphotransferase reaction. Biochim. Biophys. Acta, 793, 346-353.
Nagiec, M.M., Nagiec, E.E., Baltisberger, J.A., Wells, G.B., Lester, R.L. and Dickson, R.C. (1997) Sphingolipid synthesis as a target for antifungal drugs. J. Biol. Chem., 272, 9809-9817.
Neuwald, A.F. (1997) An unexpected structural relationship between integral membrane phosphatases and soluble haloperoxidases. Protein Sci., 6, 1764-1767.
Pettus, B.J., Chalfant, C.E. and Hannun, Y.A. (2002) Ceramide in apoptosis: an overview and current perspectives. Biochim. Biophys. Acta, 1585, 114-125. Riboni, L., Viani, P., Bassi, R., Giussani, P. and Tettamanti, G. (2001) Basic fibroblast growth factor-induced proliferation of primary astrocytes. J. Biol. Chem., 276, 12797-12804.
Rietveld, A., Neutz, S., Simons, K. and Eaton, S. (1999) Association of sterol-and glycosylphosphatidylinositol-linked proteins with Drosophila raft lipid microdomains. J. Biol. Chem., 274, 12049-12054.
Satouchi, K., Hirano, K., Sakaguchi, M., Takehara, H. and Matsuura, F. (1993) Phospholipids from the free-living nematode Caenorhabditis elegans. Lipids, 28, 837-840.
Schultz, J., Ponting, C.P., Hofmann, K. and Bork, P. (1997) SAM as a protein interaction domain involved in developmental regulation. Protein Sci, 6, 249-253.
Simons, K. and Toomre, D. (2000) Lipid rafts and signal transduction. Nat. Rev. Mol. Cell Biol., 1, 31-39.
Sprong H., Degroote, S., Claessens T., van Drunen, J., Oorschot, V., Westerink, B.H., Hirabayashi, Y., Klumperman, J., van der Sluijs, P. and van Meer, G. (2001) Glysosphingolipids are required for sorting melanosomal proteins in the Golgi complex. J. Cell Biol., 155, 369 - 380.
Trotter, P.J. (2000) A novel pathway for transport and metabolism of a fluorescent phosphatidic acid analog in yeast. Traffic, 1, 425-434.
Ullman, M.D. and Radin, N.S. (1974) The enzymatic formation of sphingomyelin from ceramide and lecithin in mouse liver. J. Biol. Chem., 249, 1506-1512.
van Helvoort, A., Stoorvogel, W., van Meer, G. and Burger, K.N.J. (1997) Sphingomyelin synthase is absent from endosomes. J. Cell Sci., 110, 781-788. van Helvoort, A., van't Hof, W., Ritsema, T., Sandra, A. and van Meer, G. (1994) Conversion of diacylglycerol to phosphatidylcholine on the basolateral surface of epithelial MDCK cells. J. Biol. Chem., 269, 1763-1769.
Voelker, D.R. and Kennedy, E.P. (1982) Cellular and enzymic synthesis of sphingomyelin. Biochem., 21, 2753-2759.
Waggoner, D.W., Xu, J., Singh, I., Jasinska, R., Zhang, Q.X. and Brindley, D.N. (1999) Structural organization of mammalian lipid phosphate phosphatases: implications for signal transduction. Biochim. Biophys. Acta, 1439, 299-316.

### Fig. 9

### Sequence listings

Sequence listings 1 - 11: conserved sequence motifs of phosphotransferases as indicated in Figure 8B.
Sequence listing 1: parial polypeptide sequences of *hs*SMS1
Sequence listing 2: parial polypeptide sequences of *hs*SMS2
Sequence listing 3: parial polypeptide sequences of *ce*SMS1
Sequence listing 4: parial polypeptide sequences of *ce*SMS2
Sequence listing 5: parial polypeptide sequences of *ce*SMS3
Sequence listing 6: parial polypeptide sequences of *pf*SMS1
Sequence listing 7: parial polypeptide sequences of *pf*SMS2
Sequence listing 8: parial polypeptide sequences of *hs*SMSr
Sequence listing 9: parial polypeptide sequences of *dm*SMSr
Sequence listing 10: parial polypeptide sequences of *ce*SMSr
Sequence listing 11: parial polypeptide sequences of *ce*SMSdr

Sequence listing 12 (SEQ ID NO: 12): *hs*SMS1

MKEVVYWSPKKVADWLLENAMPEYCEPLEHFTGQDLINLTQEDFKKPPLCRVSSDN
GQRLLDMIETLKMEHHLEAHKNGHANGHLNIGVDIPTPDGSFSIKIKPNGMPNGYRK
EMIKIPMPELERSQYPMEWGKTFLAFLYALSCFVLTTVMISVVHERVPPKEVQPPLPD
TFFDHFNRVQWAFSICEINGMILVGLWLIQWLLLKYKSIISRRFFCIVGTLYLYRCITM
YVTTLPVPGMHFNCSPKLFGDWEAQLRRIMKLIAGGGLSITGSHNMCGDYLYSGHTV
MLTLTYLFIKEYSPRRLWWYHWICWLLSVVGIFCILLAHDHYTVDVVVAYYITTRLF
WWYHTMANQQVLKEASQMNLLARVWWYRPFQYFEKNVQGIVPRSYHWPFPWPVV
HLSRQVKYSRLVNDT

Sequence listing 13 (SEQ ID NO: 13): *hs*SMS2

MDIIETAKLEEHLENQPSDPTNTYARPAEPVEEENKNGNGKPKSLSSGLRKGTKKYPD
YIQIAMPTESRNKFPLEWWKTGIAFIYAVFNLVLTTVMITVVHERVPPKELSPPLPDKF
FDYIDRVKWAFSVSEINGIILVGLWITQWLFLRYKSIVGRRFCFIIGTLYLYRCITMYVT
TLPVPGMHFQCAPKLNGDSQAKVQRILRLISGGGLSITGSHILCGDFLFSGHTVTLTLT
YLFIKEYSPRHFWWYHLICWLLSAAGIICILVAHEHYTIDVIIAYYITTRLFWWYHSMA
NEKNLKVSSQTNFLSRAWWFPIFYFFEKNVQGSIPCCFSWPLSWPPGCFKSSCKKYSR
VQKIGEDNEKST

Sequence listing 14 (SEQ ID NO: 14): *ce*SMS1

MKMSWNHQYTNYGSIADDNGDEEKAENSEGAAAEKVEKQHDDDGVVVHEETDGV
ASSRSSHHDKQKPGETKKSGDGKMDDDDIITTARSSSRRICGSAASSSDSETADDAPL
LPDEGPSHAVRLEMPGDKPASPHDRFPKTPLKTLVAFLMLVVAAAGNTITLSWIHER
YPLTPPLPDIVFELIPKIPWGLRLCENLMIGSFVSLLVLILFHRHRWIVLRRLCFIGSILY
GMRCITMMVTPVPKADEDFECSPRFGENATFSLIVMRGVWSMFGLGLNLFDNQKVV
LCGDYIYSGHTLVLVVSALFIGEYSPRRFYILHWLSWLVCSVGVIFLVLSHGHYTIDVI
LSYFACTRVFWAYHTQAAHPSIRLSVQNHQAKEFWFPLLRWFEGDIRRPVPRRFDCPI
SYSQTPVDYSFLPWFSFFFQSNPMMFPGAFTFYHTQNHSKSTMSLPK

Sequence listing 15 (SEQ ID NO: 15): *ce*SMS2

MTNSSEFTDVLQSRDPCVSNGIVINIDPIDPEPTPIRKEFTCEDTFHHEHHGNSEGFKTL
TAFLCLMLSAFLNFFLLTVIHDVVPRQPLPDLTFMIIPQQRWAWSVGDVLSTVSSVVA
FTIIFLHHQRWIVLRRTFLLGAIMYGLRAVILGVTFLPPSFHNRDEICQPQVNRTAMYG
MEIATRFLTYVITLGLTSGQDKILCGDLMFSGHTVVLTIMYFVQLQYTPRGLVILRYIA
APITFLGIAALVVSGGHYTMDVLIAYWLTSHVFWSYHQIFEMRKDDRPQAPLSRLW
WFWLCYWFESDVADGKLVNKWNWPLEGPQRMHTIMNRINYKLQ

Sequence listing 16 (SEQ ID NO: 16): *ce*SMS3

MGSVSKTVISARGASPDDEQNGTKNGISNGSEWAKCIFLFFFLFIAGMSNWAVLAYT
HDYVPRESLPDIVFSLVSEQRWASSLGDFCVALCIVMLGALLVIHQHRGTILKRVVFC
AGTLYAMRSVTLAATQLPSGYTDNQGRCRDQVESEAGVFFGRLFEQTIRIGFQSKDQ
MLCGDLLFSGHTLVMVTCSLAVAYYLPKSIKPLQWVSHVACLIGMICMTISRTHYTID
VVIAYWLSNMVFRMYHAYCEVDMCMERRKSILYSWWPCRIVDWLEQDIVPGRLEN
RCQLPWRRSTPRGQERGGASAESSDSSVTMCDNITTSHHQKHVSISSSSTYPLPC

Sequence listing 17 ((SEQ ID NO: 17): *pf*SMS1

MIEGNINANTTHQNTTNNKIEKNFIKEKDIINNDTIKILIEHNNSFNDESTNYITTFNDIK
DKHYNHKDSYSDNKGKIKTLRSNFSDIQDEVIITNIGHYFEGQENIKKSVEIKNCPYKK
KMLKIFLRRLVCATIIGIIGICIQCYFIILSDTYYKTGDEPLKDRLHEIFKEIPAFMNTPFV
NGSIMFFLAITLLRFGLFCPLLLSITILIRIILMLSFIYCIRSFFIYVTTLPCPIPTCQPLKHK
TLVENLYTFYLIITAQVYECTDLVISGHTAFTTLLTFFWFFYERNIYVKTTIFLYSIYIYII
IIISRFHYTVDVLMGYVFGGSVFLFYHYLVDVAARRYALNTSVFPQTYGFSGSFTDRF
QVFQYFIRAITYLEALDHRMNFALSYDKEWNCFCSCVPVNKNSLLIKKKNVRNEEYY
DFSDHFYHSYAGNGTYNLSTIRNIIKEFKRFFGMNKKN

Sequence listing 18 (SEQ ID NO: 18): *pf*SMS2

MNKYEYFYSPIQLLHLILLLRPILYENYTYDENDKSSIGTDMYEINMNRKMSNISISRN
STINEEEILSEYRLCKILLIKLMFALLFLLIALIIQGFFMIYSDSYYKSNTQPLSDRIHDLF
GNPPKWISYKLSNTLIAILTLSFLKIILFNSIYLSIAIICRFLYIVGSFYIIRGLLIYVTSLPA
TLETCLPLESGNFLFNLLQIIKINTNLVYVCADLIVSGHSFSTTIFLMFSFYYINNVIIKFII
FTFSCFIYAIIIIGFIHYTSDVLLGIIFGVFMFSFYHIMLDISSQYYIFNKLFEIKIISNNKNI
HAKPFFLRFFVARIFFKIIPYLEGLNYTLDYAINKNNDLSTFCNCDHDNNKIPLFSFYKP
ITEDKIIINYSDHLYHSYAGDGTINFLFWKFLKTIKKGLHK

Sequence listing 19 (SEQ ID NO: 19): *hs*SMSr

MAGPNQLCIRRWTTKHVAVWLKDEGFFEYVDILCNKHRLDGITLLTLTEYDLRSPPL
EIKVLGDIKRLMLSVRKLQKIHIDVLEEMGYNSDSPMGSMTPFISALQSTDWLCNGEL
SHDCDGPITDLNSDQYQYMNGKNKHSVRRLDPEYWKTILSCIYVFIVFGFTSFIMVIV
HERVPDMQTYPPLPDIFLDSVPRIPWAFAMTEVCGMILCYIWLLVLLLHKHRSILLRR
LCSLMGTVFLLRCFTMFVTSLSVPGQHLQCTGKIYGSVWEKLHRAFAIWSGFGMTLT
GVHTCGDYMFSGHTVVLTMLNFFVTEYTPRSWNFLHTLSWVLNLFGIFFILAAHEHY
SIDVFIAFYITTRLFLYYHTLANTRAYQQSRRARIWFPMFSFFECNVNGTVPNEYCWP
FSKPAIMKRLIG

Sequence listing 20 (SEQ ID NO: 20): *dm*SMSr

MCDGEIGDPVTQPRSEGGGLVTMDQETRTHYLDAATDKHLTNGSPDPEPVDPLLVA
QWSIENVTSWATCMEHFSRTLLDCLRQEAIDGEVLLSLTEEDVRDMRYKLGYKLTFG[?]
ELKKFWIAVLKLQLLVKNSSAESVILGIECHGNGNSVYMPLASTGCGPPSSSTCPCPQ
AECPSYVSDCDTYLRMGGRYVPPEYFKTAMSLGYSFVVTWITSLTMVIVHERVPDM
KRYPPLPDIFLDNVPHIPWAFNMCEITGSLLFTIWVVVLTFHKYRLVLLRRFFALAGT
VFLLRCVTMLITSLSVPGTHLQCSQKDFAIDDPNVDMVGALIIRMSRAYRIWSGLGMS
IQGVRTCGDYMFSGHTVALTLLNFFITEYTPRNLYFLHTLTWLLNMFGIFFILAAHEH
YSIDVFVAFYITSRLFLYYHTLANNRALMQSDSKRTRVWFPMFSYFESSVDGMVPNE
YDTLGSLIDGIIEQIFKAKDQLAISVKRCWLDAPLSGNSSAHMFGGESEQCLRNGTAS
AAFFSPHQSLIGGLGGQRSQTHLNSAQSGSSTPTPATSAPTKSLPTQKKTFRDASVDPF
SRTTFAAVQQAENVKDSPMKEKKHL

Sequence listing 21 (SEQ ID NO: 21): *ce*SMSr

MLDNRPIPADPNEWRCEDVGNWLKKIGMAKYADLIAMKHKVDGKCLLALTDTDLK
DPPVSINCLGDIKKILFAIEFLSQKVVEIGNSGVHHRSTPNGNGPSLKNSKDGLLVEYN
EQNHLSISGEDVYTTTRRAEIVEDEETLLDTLAKSSDGTSTVQLISREEIIRQVERPDTY
FKSVAKLLIAFAYSSLSFLMTSFVMVLVHDRVPDTKTYPPLPDIVLDNVPHIPWAFDM

CETIGLVLAVVWFTVLFFHNQRVIVARRMFSLLGTVFLLRCFTMLITSLSVPGIHLQCE
ARPNTTMQEKLHKAFHIWSNLGMSLHGVRSCGDYMFSGHTTVITMISHFITEYTPAD
WTGLHTFTWVLNCFAIFLILAAHEHYSIDVFIAFYISSRMFLYYHAYAYNHAGITATD
YRMRTWFPLGWFFEYGSQGKVENEFSLPINIRIPRRVFFAKSEEPKITPKSDSSRKRSS
VVAAKQNGNSKNHTKKHN

Sequence listing 22 (SEQ ID NO: 22): *ce*SMSdr

MCKSCENYGKFFFAMLKICIDNSSNFAMKKDAENENLLHAYEHDEQSSKYFIKPDSR
YSIDDDISVKIDGKQLEVRKWPTLLATAMVGVGWLSNEVALAWVHERVPDDYHPLP
DLFFSHFPEVKERLKIFGILGKFEKSEKKTSEFSSPIRGAIRIAEYIMMILLISALLVMFT
HQHRWIVIRRVFFCIAMAYSFRALCVTIFQVPVPSINTYCAPKSNSSLELVAGRVVKM
FWSAGIEQLRPRELCGDLIVSGHTLTIFTAFLVFKTYAPQRLQPLSHIYHVLAFTALFSI
LLARKHYMIDIVLGYTVSTRIFMEYHALAASYHNRTFETNPLAWSFWSFFIPIFECDAP
ANMHNHLLLYNRSTSSKNVSTLKKSRRSFE

## Claims

1. An isolated polypeptide with sphingomyelin synthase activity, ethanolamine phosphorylceramide synthase activity, phosphatidylcholine:glycoprotein cholinephosphotransferase activity or phosphatidylcholine:glycolipid cholinephosphotransferase activity, said polypeptide comprising the amino acid motifs:
(a) P-L-X-D-X(35-75)-R-R-X(8)-[YF]-X(2)-R-X(6)-T;
(b) C-X-D-X(3)-S-G-H-T; and
(c) H-Y-[TS]-X-D-[VI]-X(3)-[FYI]-X(6)-F-X(2)-Y-H,
whereby the polypeptide does not comprise an amino acid sequence selected from SEQ ID No 12, 13, 15, 16, 19, 21 and 22 as depicted in Figure 9.

2. A polypeptide according to claim 1 that is derived from the group consisting of Animalia, Alveolata and Kinetoplastida.

3. A polypeptide according to claim 1 or 2 comprising an amino acid sequence with at least 90% sequence identity to any one of SEQ ID No. 14, 17, 18 and 20 as depicted in Figure 9.

4. A polypeptide according to claim 1 or 2 comprising an amino acid sequence of any one of SEQ ID No. 14, 17, 18 and 20 as depicted in Figure 9.

5. A polypeptide according to claim 4 comprising an amino acid sequence of the SEQ ID No. 14 as depicted in Figure 9.

6. A polypeptide according to claim 4 comprising an amino acid sequence of SEQ ID No. 17 as depicted in Figure 9.

7. A polypeptide according to claim 4 comprising an amino acid sequence of the SEQ ID No. 18 as depicted in Figure 9.

8. A polypeptide according to claim 4 comprising an amino acid sequence of the SEQ ID No. 20 as depicted in Figure 9.

9. A vector or plasmid comprising a nucleotide sequence selected from the group consisting of a nucleotide sequence coding for any of the amino acid sequences as described in any one of claims 1-8 and an antisense nucleotide sequence that is complementary thereto.

10. An expression cassette comprising a nucleotide sequence selected from the group consisting of a nucleotide sequence coding for any of the amino acid sequences described in any of the claims 1-8 and an antisense nucleotide sequence that is complementary thereto.

11. A (micro)organism or cell line in which any of the nucleotide sequences selected from the group consisting of a nucleotide sequence coding for any of the amino acid sequences as described in any one of claims 1-8 and an antisense nucleotide sequence that is complementary thereto, was introduced.

12. A process for producing sphingomyelin synthase comprising the expression a nucleotide sequence coding for a polypeptide comprising the amino acid:
(a) P-L-X-D-X(35-75)-R-R-X(8)-[YF]-X(2)-R-X(6)-T;
(b) C-X-D-X(3)-S-G-H-T; and
(c) H-Y-[TS]-X-D-[VI]-X(3)-[FYI]-X(6)-F-X(2)-Y-H
in a (micro)organism or cell line of claim 11 and the isolation of sphingomyelin synthase.

13. A process for producing sphingomyelin comprising the expression a nucleotide sequence coding for a polypeptide comprising the amino acid motifs:
(a) P-L-X-D-X(35-75)-R-R-X(8)-[YF]-X(2)-R-X(6)-T;
(b) C-X-D-X(3)-S-G-H-T; and
(c) H-Y-[TS]-X-D-[VI]-X(3)-[FYI]-X(6)-F-X(2)-Y-H
in a (micro)organism or cell of claim 11 and the isolation of sphingomyelin.

14. Use of one or more of the nucleotide sequences coding for a polypeptide comprising the amino acid motifs:
(a) P-L-X-D-X(35-75)-R-R-X(8)-[YF]-X(2)-R-X(6)-T;
(b) C-X-D-X(3)-S-G-H-T; and
(c) H-Y-[TS]-X-D-[VI]-X(3)-[FYI]-X(6)-F-X(2)-Y-H
or an antisense nucleotide sequence thereto to influence the reaction
CER + PC ↔ SM + DAG
*in vitro.*

15. Use of one or more of the nucleotide sequences coding for a polypeptide comprising the amino acid motifs:
(a) P-L-X-D-X(35-75)-R-R-X(8)-[YF]-X(2)-R-X(6)-T;
(b) C-X-D-X(3)-S-G-H-T; and
(c) H-Y-[TS]-X-D-[VI]-X(3)-[FYI]-X(6)-F-X(2)-Y-H
or an antisense nucleotide sequence thereto to identify or develop compounds influencing the reaction
CER + PC ↔ SM + DAG
in vitro.

16. A process for producing ethanolamine phosphorylceramide synthase comprising the expression of a nucleotide sequence coding for a polypeptide comprising the amino acid motifs:
(a) P-L-X-D-X(35-75)-R-R-X(8)-[YF]-X(2)-R-X(6)-T;
(b) C-X-D-X(3)-S-G-H-T; and
(c) H-Y-[TS]-X-D-[VI]-X(3)-[FYI]-X(6)-F-X(2)-Y-H
in a (micro)organism or cell line of claim 11 and the isolation of ethanolamine phosphorylceramide synthase.

17. A process for producing ethanolamine phosphorylceramide comprising the expression of the nucleotide sequence coding for a polypeptide comprising the amino acid motifs:
(a) P-L-X-D-X(35-75)-R-R-X(8)-[YF]-X(2)-R-X(6)-T;
(b) C-X-D-X(3)-S-G-H-T; and
(c) H-Y-[TS]-X-D-[VI]-X(3)-[FYI]-X(6)-F-X(2)-Y-H
in a (micro)organism or cell line of claim 11 and the isolation of ethanolamine phosphorylceramide.

18. Use of any one of the nucleotide sequences coding for a polypeptide comprising the amino acid motifs:
(a) P-L-X-D-X(35-75)-R-R-X(8)-[YF]-X(2)-R-X(6)-T;
(b) C-X-D-X(3)-S-G-H-T; and
(c) H-Y-[TS]-X-D-[VI]-X(3)-[F'YI]-X(6)-F-X(2)-Y-H
or an antisense nucleotide sequence thereto, to influence the reaction
CER + PE ↔ EPC + DAG
*in vitro.*

19. Use of a nucleotide sequence coding for a polypeptide comprising the amino acid motifs:
(a) P-L-X-D-X(35-75)-R-R-X(8)-[YF]-X(2)-R-X(6)-T;
(b) C-X-D-X(3)-S-G-H-T; and
(c) H-Y-[TS]-X-D-[VI]-X(3)-[FY'I]-X(6)-F-X(2)-Y-H
or an antisense nucleotide sequence thereto to identify or develop compounds influencing the reaction
CER + PE ↔ EPC + DAG
*in vitro.*

20. A process for producing phosphatidyl:glycoprotein cholinephosphotransferase or phosphatidyl:glycolipid cholinephosphotransferase comprising the expression of a corresponding nucleotide sequence coding for a polypeptide comprising the amino acid motifs:
(a) P-L-X-D-X(35-75)-R-R-X(8)-[YF]-X(2)-R-X(6)-T;
(b) C-X-D-X(3)-S-G-H-T; and
(c) H-Y-[TS]-X-D-[VI]-X(3)-[FYI]-X(6)-F-X(2)-Y-H
in a (micro)organism or a cell line of claim 11 and the isolation of phosphatidyl:glycoprotein cholinephosphotransferase or phosphatidyl:glycolipid cholinephosphotransferase.

21. A process for producing phosphorylcholine-substituted glycoprotein or phosphorylcholine-substituted glycolipid comprising the expression of a corresponding nucleotide sequence coding for a polypeptide comprising the amino acid motifs:
(a) P-L-X-D-X(35-75)-R-R-X(8)-[YF]-X(2)-R-X(6)-T;
(b) C-X-D-X(3)-S-G-H-T; and
(c) H-Y-[TS]-X-D-[VI]-X(3)-[FYI]-X(6)-F-X(2)-Y-H
in a (micro)organism or cell of claim 11 and the isolation of phosphorylcholine-substituted glycoprotein or phosphorylcholine-substituted glycolipid.

22. Use of one or more of the nucleotide sequences coding for a polypeptide comprising the amino acid motifs:
(a) P-L-X-D-X(35-75)-R-R-X(8)-[YF]-X(2)-R-X(6)-T;
(b) C-X-D-X(3)-S-G-H-T; and
(c) H-Y-[TS]-X-D-[VI]-X(3)-[FYI]-X(6)-F-X(2)-Y-H
or an antisense nucleotide sequence that is complementary thereto to influence the reaction
glyco lipid/protein + PC ↔ PC-substituted glyco lipid/protein + DAG
*in vitro.*

23. Use of one or more of the nucleotide sequences coding for a polypeptide comprising the amino acid motifs:
(a) P-L-X-D-X(35-75)-R-R-X(8)-[YF]-X(2)-R-X(6)-T;
(b) C-X-D-X(3)-S-G-H-T; and
(c) H-Y-[TS]-X-D-[VI]-X(3)-[FYI]-X(6)-F-X(2)-Y-H
or an antisense nucleotide sequence that is complementary thereto to identify or develop compounds influencing the reaction
glyco lipid/protein + PC ↔ PC-substituted glyco lipid/protein + DAG
*in vitro*.

24. A method for determining whether a compound is capable of lowering or upregulating an enzymatic activity displayed by a cell, said activity comprising an activity of an enzyme of the group of enzymes identified as sphingomyelin synthases, ethanolamine phosphorylceramide synthases, phosphatidylcholine:glycoprotein cholinephosphotransferase and phosphatidylcholine:glycolipid cholinephosphotransferase, said method comprising providing said cell with a nucleic acid encoding a polypeptide comprising the amino acid motifs:
(a) P-L-X-D-X(35-75)-R-R-X(8)-[YF]-X(2)-R-X(6)-T;
(b) C-X-D-X(3)-S-G-H-T; and
(c) H-Y-[TS]-X-D-[VI]-X(3)-[FYI]-X(6)-F-X(2)-Y-H
contacting said cell with said compound and determining whether said enzymatic activity is lowered or upregulated.

25. A method according to claim 24 wherein said cell is deficient in sphingomyelin synthase activity.

26. A method according to claim 24 or claim 25, wherein said cell is a cell of a eukaryotic micro-organism.

27. A method according to claim 26, wherein said cell is yeast cell.

28. A method according to any one of claims 24-27, wherein said polypeptide comprises an amino acid sequence with at least 90% sequence identity to any one of SEQ ID No. 12-22, as depicted in Figure 9.

29. A method according to any one of claims 24-28, wherein said polypeptide is derived from a plasmodium.

30. A method according to any one of claims 24-29, wherein said compound comprises RNA.

31. Use of an oligonucleotide specific for a nucleic acid sequence encoding a polypeptide comprising the amino acid motifs:
(a) P-L-X-D-X(35-75)-R-R-X(8)-[YF]-X(2)-R-X(6)-T;
(b) C-X-D-X(3)-S-G-H-T; and
(c) H-Y-[TS]-X-D-[VI]-X(3)-[FYI]-X(6)-F-X(2)-Y-H
for detecting said sequence,
whereby the polypeptide does not comprise an amino acid sequence selected from SEQ ID No 12, 13, 15, 16, 19, 21 and 22 as depicted in Figure 9.

32. Use according to claim 31, for assessing whether a cell comprises sphingomyelin synthase activity.

33. An inhibitor of a polypeptide with sphingomyelin synthase comprising the amino acid motifs:
(a) P-L-X-D-X(35-75)-R-R-X(8)-[YF]-X(2)-R-X(6)-T;
(b) C-X-D-X(3)-S-G-H-T; and
(c) H-Y-[TS]-X-D-[VI]-X(3)-[FYI]-X(6)-F-X(2)-Y-H
for use as a cell death promoter.

34. An inhibitor of a polypeptide with sphingomyelin synthase activity comprising the amino acid motifs:
(a) P-L-X-D-X(35-75)-R-R-X(8)-[YF]-X(2)-R-X(6)-T;
(b) C-X-D-X(3)-S-G-H-T; and
(c) H-Y-[TS]-X-D-[VI]-X(3)-[FYI]-X(6)-F-X(2)-Y-H
for use to promote cell death.

35. An inhibitor according to claim 33 or 34, wherein said cell is a cell of a parasite.

36. An inhibitor according to claim 33 or 34, wherein said cell is a human cell, preferably a tumor cell.

37. A nucleic acid coding for a polypeptide comprising the amino acid motifs:
(a) P-L-X-D-X(35-75)-R-R-X(8)-[YF]-X(2)-R-X(6)-T;
(b) C-X-D-X(3)-S-G-H-T; and
(c) H-Y-[TS]-X-D-[VI]-X(3)-[FYI]-X(6)-F-X(2)-Y-H
for use in enhancing cell survival and/or cell growth.

38. A method for improving the yield of a secretion product of a cell comprising providing said cell with a polypeptide comprising the amino acid motifs:
(a) P-L-X-D-X(35-75)-R-R-X(8)-[YF]-X(2)-R-X(6)-T;
(b) C-X-D-X(3)-S-G-H-T; and
(c) H-Y-[TS]-X-D-[VI]-X(3)-[FYI]-X(6)-F-X(2)-Y-H
or a nucleic acid coding for said polypeptide.

39. A method according to claim 38, comprising providing said cell with a polypeptide comprising an amino acid sequence with at least 90% sequence identity to any one of SEQ ID No. 12-22, as depicted in Figure 9, or a nucleic acid coding for a polypeptide comprising an amino acid sequence with at least 90% sequence identity to any one of SEQ ID No. 12-22, as depicted in Figure 9.

40. A method according to claim 38 or 39, wherein said cell is a cell of a eukaryotic micro-organism.

41. A method according to any one of claims 24-30, further comprising providing said cell or a fraction thereof with a labelled substrate for said sphingomyelin synthase.

42. A method according to claim 41, further comprising harvesting sphingolipid from said cell or said fraction and detecting labelled sphingolipid.

43. A method according to claim 41 or 42, further comprising detecting said labelled sphingolipid using (thin layer) chromatography or mass spectrometry.

44. A method for targeting a first polypeptide comprising the amino acid motifs:
(a) P-L-X-D-X(35-75)-R-R-X(8)-[YF]-X(2)-R-X(6)-T;
(b) C-X-D-X(3)-S-G-H-T; and
(c) H-Y-[TS]-X-D-[VI]-X(3)-[FYI]-X(6)-F-X(2)-Y-H
to a different cellular compartment comprising providing a cytosolic part of said first polypeptide with a cellular compartment localization signal of a cytosolic part of a second polypeptide comprising the amino acid motifs:
(a) P-L-X-D-X(35-75)-R-R-X(8)-[YF]-X(2)-R-X(6)-T;
(b) C-X-D-X(3)-S-G-H-T; and
(c) H-Y-[TS]-X-D-[VI]-X(3)-[FYI]-X(6)-F-X(2)-Y-H
wherein said first and said second polypeptide, when unmodified, reside in different cellular compartments.

45. A method according to claim 44, wherein said cytosolic part of said first polypeptide comprises the C-terminal end of said polypeptide.

46. A method according to claim 44 or claim 45, wherein said cytosolic part of said second polypeptide comprises the C-terminal end of said polypeptide.

47. A method according to any one of claims 44-46, wherein said cellular compartments comprises the plasma membrane, the endosomal compartment, the Golgi, the endoplasmatic reticulum or a combination thereof.

48. A method according to any one of claims 44-47, wherein said cellular compartment localization signal of said second polypeptide replace the C-terminal cytosolic part of said first polypeptide.

## Patentansprüche

1. Isoliertes Polypeptid mit Sphingomyelin-Synthaseaktivität, Ethanolaminphosphorylceramid-Synthaseaktivität, Phosphatidylcholin:Glykoprotein-Cholinphospho-Transferaseaktivität oder Phosphatidylcholin:Glykolipid-Cholinphospho-Transferaseaktivität, wobei das Polypeptid folgende Aminosäuremotive umfasst:
(a) P-L-X-D-X(35-75)-R-R-X(8)-[YF]-X(2)-R-X(6)-T;
(b) C-X-D-X(3)-S-G-H-T; und
(c) H-Y-[TS]-X-D-[VI]-X(3)-[FYI]-X(6)-F-X(2)-Y-H,
wobei das Polypeptid keine Aminosäuresequenz ausgewählt aus SEQ ID NO: 12, 13, 15, 16, 19, 21 und 22, wie in Fig. 9 dargestellt, umfasst.

2. Polypeptid nach Anspruch 1, das aus der Gruppe, bestehend aus Animalia, Alveolata und Kinetoplastida, stammt.

3. Polypeptid nach Anspruch 1 oder 2, umfassend eine Aminosäuresequenz mit mindestens 90 % Sequenzidentität mit einer der SEQ ID NO: 14, 17, 18 und 20 wie in Fig. 9 dargestellt.

4. Polypeptid nach Anspruch 1 oder 2, umfassend eine Aminosäuresequenz einer der SEQ ID NO: 14, 17, 18 und 20 wie in Fig. 9 dargestellt.

5. Polypeptid nach Anspruch 4, umfassend eine Aminosäuresequenz der SEQ ID NO: 14 wie in Fig. 9 dargestellt.

6. Polypeptid nach Anspruch 4, umfassend eine Aminosäuresequenz der SEQ ID NO: 17 wie in Fig. 9 dargestellt.

7. Polypeptid nach Anspruch 4, umfassend eine Aminosäuresequenz der SEQ ID NO: 18 wie in Fig. 9 dargestellt.

8. Polypeptid nach Anspruch 4, umfassend eine Aminosäuresequenz der SEQ ID NO: 20 wie in Fig. 9 dargestellt.

9. Vektor oder Plasmid, umfassend eine Nukleotidsequenz, ausgewählt aus der Gruppe bestehend aus einer Nukleotidsequenz kodierend irgendeine der Aminosäuresequenzen wie in einem der Ansprüche 1-8 beschrieben, und einer Antisense-Nukleotidsequenz, die komplementär dazu ist.

10. Expressionskassette, umfassend eine Nukleotidsequenz, ausgewählt aus der Gruppe bestehend aus einer Nukleotidsequenz kodierend firgendeine der Aminosäuresequenzen wie in einem der Ansprüche 1-8 beschrieben, und einer Antisense-Nukleotidsequenz, die komplementär dazu ist.

11. (Mikro)organismus oder Zelllinie, in den bzw. die eine der Nukleotidsequenzen, ausgewählt aus der Gruppe bestehend aus einer Nukleotidsequenz kodierend irgendeine der Aminosäuresequenzen, wie in einem der Ansprüche 1-8 beschrieben, und einer Antisense-Nukleotidsequenz, die komplementär dazu ist, eingebracht wurde.

12. Verfahren zur Herstellung von Sphingomyelin-Synthase, umfassend die Expression einer Nukleotidsequenz kodierend ein Polypeptid, umfassend die Aminosäure:
(a) P-L-X-D-X(35-75)-R-R-X(8)-[YF]-X(2)-R-X(6)-T;
(b) C-X-D-X(3)-S-G-H-T; und
(c) H-Y-[TS]-X-D-[VI]-X(3)-[FYI]-X(6)-F-X(2)-Y-H,
in einem (Mikro)organismus oder einer Zelllinie nach Anspruch 11 und Isolieren der Sphingomyelin-Synthase.

13. Verfahren zur Herstellung von Sphingomyelin, umfassend die Expression einer Nukleotidsequenz kodierend ein Polypeptid, umfassend die Aminosäuremotive:
(a) P-L-X-D-X(35-75)-R-R-X(8)-[YF]-X(2)-R-X(6)-T;
(b) C-X-D-X(3)-S-G-H-T; und
(c) H-Y-[TS]-X-D-[VI]-X(3)-[FYI]-X(6)-F-X(2)-Y-H,
in einem (Mikro)organismus oder einer Zelle nach Anspruch 11 und Isoloieren von Sphingomyelin.

14. Verwendung einer oder mehrerer der Nukleotidsequenz kodierend ein Polypeptid, umfassend die Aminosäuremotive:
(a) P-L-X-D-X(35-75)-R-R-X(8)-[YF]-X(2)-R-X(6)-T;
(b) C-X-D-X(3)-S-G-H-T; und
(c) H-Y-[TS]-X-D-[VI]-X(3)-[FYI]-X(6)-F-X(2)-Y-H,
oder eine Antisense-Nukleotidsequenz dazu, zur Beeinflussung der Reaktion
CER + PC ↔ SM + DAG
*in vitro.*

15. Verwendung einer oder mehrerer der Nukleotidsequenzen kodierend für ein Polypeptid, umfassend die Aminosäuremotive:
(a) P-L-X-D-X(35-75)-R-R-X(8)-[YF]-X(2)-R-X(6)-T;
(b) C-X-D-X(3)-S-G-H-T; und
(c) H-Y-[TS]-X-D-[VI]-X(3)-[FYI]-X(6)-F-X(2)-Y-H,
oder eine Antisense-Nukleotidsequenz dazu, zur Identifizierung oder Entwicklung von Verbindungen, die die Reaktion
CER + PC ↔ SM + DAG
*in vitro* beeinflussen.

16. Verfahren zur Herstellung einer Ethanolaminphosphorylceramid-Synthase, umfassend die Expression einer Nukleotidsequenz kodierend ein Polypeptid, umfassend die Aminosäuremotive:
(a) P-L-X-D-X(35-75)-R-R-X(8)-[YF]-X(2)-R-X(6)-T;
(b) C-X-D-X(3)-S-G-H-T; und
(c) H-Y-[TS]-X-D-[VI]-X(3)-[FYI]-X(6)-F-X(2)-Y-H,
in einem (Mikro)organismus oder einer Zelllinie nach Anspruch 11 und Isolieren von Ethanolaminphosphorylceramid-Synthase.

17. Verfahren zur Herstellung von Ethanolaminphosphorylceramid, umfassend die Expression der Nukleotidsequenz kodierend ein Polypeptid, umfassend die Aminosäuremotive:
(a) P-L-X-D-X(35-75)-R-R-X(8)-[YF]-X(2)-R-X(6)-T;
(b) C-X-D-X(3)-S-G-H-T; und
(c) H-Y-[TS]-X-D-[VI]-X(3)-[FYI]-X(6)-F-X(2)-Y-H,
in einem (Mikro)organismus oder einer Zelllinie nach Anspruch 11 und Isolieren von Ethanolaminphosphorylceramid.

18. Verwendung einer der Nukleotidsequenzen kodierend ein Polypeptid, umfassend die Aminosäuremotive:
(a) P-L-X-D-X(35-75)-R-R-X(8)-[YF]-X(2)-R-X(6)-T;
(b) C-X-D-X(3)-S-G-H-T; und
(c) H-Y-[TS]-X-D-[VI]-X(3)-[FYI]-X(6)-F-X(2)-Y-H,
oder eine Antisense-Nukleotidsequenz dazu, zur Beeinflussung der Reaktion
CER + PE ↔ EPC + DAG
*in vitro.*

19. Verwendung einer Nukleotidsequenz kodierend ein Polypeptid, umfassend die Aminosäuremotive:
(a) P-L-X-D-X(35-75)-R-R-X(8)-[YF]-X(2)-R-X(6)-T;
(b) C-X-D-X(3)-S-G-H-T; und
(c) H-Y-[TS]-X-D-[VI]-X(3)-[FYI]-X(6)-F-X(2)-Y-H,
oder eine Antisense-Nukleotidsequenz dazu, zur Identifizierung oder Entwicklung von Verbindungen, die die Reaktion
CER + PE ↔ EPC + DAG
*in vitro* beeinflussen.

20. Verfahren zur Herstellung von Phosphatidyl:Glykoprotein-Cholinphospho-Transferase oder Phosphatidyl:Glykolipid-Cholinphospho-Transferase, umfassend die Expression einer entsprechenden Nukleotidsequenz kodierend ein Polypeptid, umfassend die Aminosäuremotive:
(a) P-L-X-D-X(35-75)-R-R-X(8)-[YF]-X(2)-R-X(6)-T;
(b) C-X-D-X(3)-S-G-H-T; und
(c) H-Y-[TS]-X-D-[VI]-X(3)-[FYI]-X(6)-F-X(2)-Y-H,
in einem (Mikro)organismus oder einer Zelllinie nach Anspruch 11 und Isoieren von Phosphatidyl:Glykoprotein-Cholinphospho-Transferase oder Phosphatidyl:Glykolipid-Cholinphospho-Transferase.

21. Verfahren zur Herstellung von Phosphorylcholin-substituiertem Glykoprotein oder Phosphorylcholin-substituiertem Glykolipid, umfassend die Expression einer entsprechenden Nukleotidsequenz kodierend ein Polypeptid, umfassend die Aminosäuremotive:
(a) P-L-X-D-X(35-75)-R-R-X(8)-[YF]-X(2)-R-X(6)-T;
(b) C-X-D-X(3)-S-G-H-T; und
(c) H-Y-[TS]-X-D-[VI]-X(3)-[FYI]-X(6)-F-X(2)-Y-H,
in einem (Mikro)organismus oder einer Zelle nach Anspruch 11 und Isolieren von Phosphorylcholin-substituiertem Glykoprotein oder Phosphorylcholin-substituiertem Glykolipid.

22. Verwendung einer oder mehrerer der Nukleotidsequenzen kodierend ein Polypeptid, umfassend die Aminosäuremotive:
(a) P-L-X-D-X(35-75)-R-R-X(8)-[YF]-X(2)-R-X(6)-T;
(b) C-X-D-X(3)-S-G-H-T; und
(c) H-Y-[TS]-X-D-[VI]-X(3)-[FYI]-X(6)-F-X(2)-Y-H,
oder eine Antisense-Nukleotidsequenz, die komplementär dazu ist, zum Beeinflussen der Reaktion
Glykolipid/Protein + PC ↔ PC-substituiertes Glykolipid/Protein + DAG
*in vitro.*

23. Verwendung einer oder mehrerer der Nukleotidsequenzen kodierend ein Polypeptid, umfassend die Aminosäuremotive:
(a) P-L-X-D-X(35-75)-R-R-X(8)-[YF]-X(2)-R-X(6)-T;
(b) C-X-D-X(3)-S-G-H-T; und
(c) H-Y-[TS]-X-D-[VI]-X(3)-[FYI]-X(6)-F-X(2)-Y-H,
oder eine Antisense-Nukleotidsequenz, die komplementär dazu ist, zur Identifizierung oder Entwicklung von Verbindungen, die die Reaktion
Glykolipid/Protein + PC ↔ PC-substituiertes Glykolipid/Protein + DAG
*in vitro* beeinflussen.

24. Verfahren zum Bestimmen, ob eine Verbindung in der Lage ist, eine von einer Zelle gezeigte enzymatische Aktivität zu senken oder hoch zu regulieren, wobei die Aktivität eine Aktivität eines Enzyms der Gruppe von Enzymen, identifiziert als Sphingomyelin-Synthasen, Ethanolaminphosphorylceramid-Synthasen, Phosphatidylcholin:Glykoprotein-Cholinphospho-Transferase und Phosphatidylcholiln:Glykolilpid-Cholinphospho-Transferase, umfasst, wobei das Verfahren das Versehen der Zelle mit einer Nukleinsäure, die ein Polypeptid codiert, das folgende Aminosäuremotive umfasst:
(a) P-L-X-D-X(35-75)-R-R-X(8)-[YF]-X(2)-R-X(6)-T;
(b) C-X-D-X(3)-S-G-H-T; und
(c) H-Y-[TS]-X-D-[VI]-X(3)-[FYI]-X(6)-F-X(2)-Y-H,
das Inberührungbringen der Zelle mit der Verbindung und bestimmen, ob die enzymatische Aktivität gesenkt oder hochreguliert wird, umfasst.

25. Verfahren nach Anspruch 24, wobei die Zelle einen Mangel an Sphingomyelin-Synthaseaktivität aufweist.

26. Verfahren nach Anspruch 24 oder 25, wobei die Zelle eine Zelle eines eukaryotischen Mikroorganismus ist.

27. Verfahren nach Anspruch 26, wobei die Zelle eine Hefezelle ist.

28. Verfahren nach einem der Ansprüche 24-27, wobei das Polypeptid eine Aminosäuresequenz mit mindestens 90 % Sequenzidentität mit einer der in Figur 9 SEQ ID NO: 12-22, wie in Fig. 9 dargestellt, aufweist.

29. Verfahren nach einem der Ansprüche 24-28, wobei das Polypeptid von einem Plasmodium stammt.

30. Verfahren nach einem der Ansprüche 24-29, wobei die Verbindung RNA umfasst.

31. Verwendung eines Oligonukleotids, spezifisch für eine Nukleinsäure, die ein Polypeptid kodiert, das die Aminosäuremotive umfasst:
(a) P-L-X-D-X(35-75)-R-R-X(8)-[YF]-X(2)-R-X(6)-T;
(b) C-X-D-X(3)-S-G-H-T; und
(c) H-Y-[TS]-X-D-[VI]-X(3)-[FYI]-X(6)-F-X(2)-Y-H,
zum Detektieren der Sequenz, wobei das Polypeptid keine Aminosäuresequenz, ausgewählt aus SEQ ID NO: 12, 13, 15, 16, 19, 21 und 22, wie in Fig. 9 dargestellt, umfasst.

32. Verwendung nach Anspruch 31, um zu beurteilen, ob eine Zelle eine Sphingomyelin-Synthaseaktivität umfasst.

33. Inhibitor eines Polypeptids mit Sphingomyelin-Synthase, umfassend die Aminosäuremotive:
(a) P-L-X-D-X(35-75)-R-R-X(8)-[YF]-X(2)-R-X(6)-T;
(b) C-X-D-X(3)-S-G-H-T; und
(c) H-Y-[TS]-X-D-[VI]-X(3)-[FYI]-X(6)-F-X(2)-Y-H,
zur Verwendung als Zelltodpromotor.

34. Inhibitor eines Polypeptids mit Sphingomyelin-Synthaseaktivität, umfassend die Aminosäuremotive:
(a) P-L-X-D-X(35-75)-R-R-X(8)-[YF]-X(2)-R-X(6)-T;
(b) C-X-D-X(3)-S-G-H-T; und
(c) H-Y-[TS]-X-D-[VI]-X(3)-[FYI]-X(6)-F-X(2)-Y-H,
zur Verwendung um den Zelltod zu begünstigen.

35. Inhibitor nach Anspruch 33 oder 34, wobei die Zelle eine Zelle eines Parasiten ist.

36. Inhibitor nach Anspruch 33 oder 34, wobei die Zelle eine humane Zelle, vorzugsweise eine Tumorzelle, ist.

37. Nukleinsäure kodierend ein Polypeptid, umfassend die Aminosäuremotive:
(a) P-L-X-D-X(35-75)-R-R-X(8)-[YF]-X(2)-R-X(6)-T;
(b) C-X-D-X(3)-S-G-H-T; und
(c) H-Y-[TS]-X-D-[VI]-X(3)-[FYI]-X(6)-F-X(2)-Y-H,
zur Verwendung bei der Verbesserung des Überlebens und/oder Wachstums von Zellen.

38. Verfahren zum Verbessern des Ertrags eines Sekretionsprodukts einer Zelle, umfassend das Versehen der Zelle mit einem Polypeptid, umfassend die Aminosäuremotive:
(a) P-L-X-D-X(35-75)-R-R-X(8)-[YF]-X(2)-R-X(6)-T;
(b) C-X-D-X(3)-S-G-H-T; und
(c) H-Y-[TS]-X-D-[VI]-X(3)-[FYI]-X(6)-F-X(2)-Y-H,
oder eine Nukleinsäure kodierend das Polypeptid.

39. Verfahren nach Anspruch 38, umfassend das Versehen der Zelle mit einem Polypeptid, umfassend eine Aminosäuresequenz mit mindestens 90 % Sequenzidentität mit einer der SEQ ID NO: 12-22, wie in Fig. 9 dargestellt, oder eine Nukleinsäure kodierend ein Polypeptid, umfassend eine Aminosäuresequenz mit mindestens 90 % Sequenzidentität zu einer der SEQ ID NO: 12-22, wie in Fig. 9 dargestellt.

40. Verfahren nach Anspruch 38 oder 39, wobei die Zelle eine Zelle eines eukaryotischen Mikroorganismus ist.

41. Verfahren nach einem der Ansprüche 24-30, ferner umfassend das Versehen der Zelle oder einer Fraktion davon mit einem markierten Substrat für die Sphingomyelin-Synthase.

42. Verfahren nach Anspruch 41, ferner umfassend das Ernten von Sphingolipid aus der Zelle oder der Fraktion und Detektieren des markierten Sphingolipids.

43. Verfahren nach Anspruch 41 oder 42, ferner umfassend das Detektieren des markierten Sphingolipids unter Verwendung von (Dünnschicht)-Chromatographie oder Massenspektrometrie.

44. Verfahren zum Targeting eines ersten Polypeptids, umfassend die Aminosäuremotive:
(a) P-L-X-D-X(35-75)-R-R-X(8)-[YF]-X(2)-R-X(6)-T;
(b) C-X-D-X(3)-S-G-H-T; und
(c) H-Y-[TS]-X-D-[VI]-X(3)-[FYI]-X(6)-F-X(2)-Y-H,
auf ein anderes Zellkompartiment, umfassend das Versehen eines zytosolischen Teils des ersten Polypeptids mit einem Zellkompartiment-Lokalisierungssignal eines zytosolischen Teils seines zweiten Polypeptids, umfassend die Aminosäuremotive:
(a) P-L-X-D-X(35-75)-R-R-X(8)-[YF]-X(2)-R-X(6)-T;
(b) C-X-D-X(3)-S-G-H-T; und
(c) H-Y-[TS]-X-D-[VI]-X(3)-[FYI]-X(6)-F-X(2)-Y-H,
wobei das erste und das zweite Polypeptid, wenn sie nicht modifiziert sind, sich in verschiedenen Zellkompartimenten befinden.

45. Verfahren nach Anspruch 44, wobei der zytosolische Teil des ersten Polypeptids das C-terminale Ende des Polypeptids umfasst.

46. Verfahren nach Anspruch 44 oder Anspruch 45, wobei der zytosolische Teil des zweiten Polypeptids das C-terminale Ende des Polypeptids umfasst.

47. Verfahren nach einem der Ansprüche 44-46, wobei die Zellkompartimente die Plasmamembran, das endosomale Kompartiment, Golgi, das endoplasmatische Retikulum oder eine Kombination davon umfassen.

48. Verfahren nach einem der Ansprüche 44-47, wobei das Zellkompartiment-Lokalisierungssignal des zweiten Polypeptids den C-terminalen zytosolischen Teil des ersten Polypeptids ersetzt.

## Revendications

1. Polypeptide isolé ayant une activité sphingomyéline synthase, une activité éthanolamine pfiosphorylcéramide synthase, une activité phosphatidylcholine : glycoprotéine cholinephosphotransférase ou une activité phosphatidylcholine : glycolipide cholinephosphotransférase, ledit polypeptide comprenant les motifs d'aminoacides :
(a) P-L-X-D-X(35-75)-R-R-X(8)-[VF]-X(2)-R-X(6)-T;
(b) C-X-D-X(3)-S-G-H-T; **et**
(c) H-Y-[TS]-X-D-[VI]-X(3)-[FYI]-X(6)-F-X(2)-Y-H,
où le polypeptide ne comprend pas de séquence d'aminoacides choisie parmi SEQ ID No 12, 13, 15, 16, 19, 21 et 22 comme représenté sur la figure 9.

2. Polypeptide selon la revendication 1 qui est dérivé du groupe consistant en Animalia, Alveolata et Kinetoplastida.

3. Polypeptide selon la revendication 1 ou 2 comprenant une séquence d'aminoacides ayant au moins 90 % d'identité de séquence avec l'une quelconque de SEQ ID No. 14, 17, 18 et 20 comme représenté sur la figure 9.

4. Polypeptide selon la revendication 1 ou 2 comprenant une séquence d'aminoacides de l'une quelconque de SEQ ID No. 14, 17, 18 et 20 comme représenté sur la figure 9.

5. Polypeptide selon la revendication 4 comprenant une séquence d'aminoacides de SEQ ID No. 14 comme représenté sur la figure 9.

6. Polypeptide selon la revendication 4 comprenant une séquence d'aminoacides de SEQ ID No. 17 comme représenté sur la figure 9.

7. Polypeptide selon la revendication 4 comprenant une séquence d'aminoacides de SEQ ID No. 18 comme représenté sur la figure 9.

8. Polypeptide selon la revendication 4 comprenant une séquence d'aminoacides de SEQ ID No. 20 comme représenté sur la figure 9.

9. Vecteur ou plasmide comprenant une séquence nucléotidique choisie dans le groupe consistant en une séquence nucléotidique codant l'une quelconque des séquences d'aminoacides comme décrit dans l'une quelconque des revendications 1-8 et une séquence nucléotidique antisens qui est complémentaire de celle-ci.

10. Cassette d'expression comprenant une séquence nucléotidique choisie dans le groupe consistant en une séquence nucléotidique codant l'une quelconque des séquences d'aminoacides décrites dans l'une quelconque des revendications 1-8 et une séquence nucléotidique antisens qui est complémentaire de celle-ci.

11. (micro)organisme ou lignée cellulaire où l'une quelconque des séquences nucléotidiques choisies dans le groupe consistant en une séquence nucléotidique codant l'une quelconque des séquences d'aminoacides comme décrit dans l'une quelconque des revendications 1-8 et une séquence nucléotidique antisens qui est complémentaire de celle-ci, a été introduite.

12. Procédé pour produire de la sphingomyéline synthase comprenant l'expression d'une séquence nucléotidique codant un polypeptide comprenant l'aminoacide :
(a) P-L-X-D-X(35-75)-**R**-R-X(8)-[YF]-X(2)-R-X(6)-T;
(b) C-X-D-X(3)-S-G-H-**T**; **et**
(c) H-Y-[TS]-X-D-[VY]-X(3)-[FYI]-X(6)-F-X(2)-Y-H
dans un (micro)organisme ou une lignée cellulaire selon la revendication 11 et l'isolement de la sphingomyéline synthase.

13. Procédé pour produire de la sphingomyéline comprenant l'expression d'une séquence nucléotidique codant un polypeptide comprenant les motifs d'aminoacides :
(a) P-L-X-D-X(35-75)-**R-R**-X(8)-[YF]-X(2)-R-X(6)-T;
(b) C-X-D-X(3)-S-G-H-T; **et**
(c) H-Y-[TS]-X-D-[VI]-X(3)-[FYI]-X(6)-F-X(2)-Y-H
dans un (micro)organisme ou une cellule selon la revendication 11 et l'isolement de la sphingomyéline.

14. Utilisation d'une ou plusieurs des séquences nucléotidiques codant un polypeptide comprenant les motifs d'aminoacides :
(a) P-L-X-D-X(35-75)-R-R-X(8)-[YF]-X(2)-R-X(6)-T;
(b) C-X-D-X(3)-S-G-H-T; **et**
(c) H-Y-[TS]-X-D-[VI]-X(3)-[FYI]-X(6)-F-X(2)-Y-H
ou d'une séquence nucléotidique antisens de celle-ci pour influencer la réaction
CER + PC ↔ SM + DAG
*in vitro.*

15. Utilisation d'une ou plusieurs des séquences nucléotidiques codant un polypeptide comprenant les motifs d'aminoacides :
(a) P-L-X-D-X(35-75)-R-R-X(8)-[YF]-X(2)-R-X(6)-T;
(b) C-X-D-X(3)-S-G-H-T; **et**
(c) H-Y-[TS]-X-D-[VI]-X(3)-[FYI]-X(6)-F-X(2)-Y-H
ou d'une séquence nucléotidique antisens de celle-ci pour identifier ou développer des composés influençant la réaction
CER + PC ↔ SM + DAG
*in vitro.*

16. Procédé pour produire de l'éthanolamine phosphorylcéramide synthase comprenant l'expression d'une séquence nucléotidique codant un polypeptide comprenant les motifs d'aminoacides:
(a) P-L-X-D-X(35-75)-R-R-X(8)-[YF]-X(2)-R-X(6)-T;
(b) C-X-D-X(3)-S-G-H-T; **et**
(c) H-Y-[TS]-X-D-[VI]-X(3)-[FYI]-X(6)-F-X(2)-Y-H
dans un (micro)organisme ou une lignée cellulaire selon la revendication 11 et l'isolement de l'éthanolamine phosphorylcéramide synthase.

17. Procédé pour produire de l'éthanolamine phosphorylcéramide comprenant l'expression d'une séquence nucléotidique codant un polypeptide comprenant les motifs aminoacides :
(a) P-L-X-D-X(35-75)-R-R-X(8)-[YF]-X(2)-R-X(6)-T;
(b) C-X-D-X(3)-S-G-H-T; **et**
(c) H-Y-[TS]-X-D-[VI]-X(3)-[FYI]-X(6)-F-X(2)-Y-H
dans un (micro)organisme ou une lignée cellulaire selon la revendication 11 et l'isolement de l'éthanolamine phosphorylcéramide.

18. Utilisation de l'une quelconque des séquences nucléotidiques codant un polypeptide comprenant les motifs d'aminoacides :
(a) P-L-X-D-X(35-75)-R-R-X(8)-[YF]-X(2)-R-X(6)-T;
(b) C-X-D-X(3)-S-G-H-T; **et**
(c) H-Y-[TS]-X-D-[VI]-X(3)-[FYI]-X(6)-F-X(2)-Y-H
ou d'une séquence nucléotidique antisens de celle-ci pour influencer la réaction
CER + PE ↔ EPC + DAG
*in vitro.*

19. Utilisation d'une séquence nucléotidique codant un polypeptide comprenant les motifs d'aminoacides :
(a) P-L-X-D-X(35-76)-R-R-X(8)-[YF]-X(2)-R-X(6)-T;
(b) C-X-D-X(3)-S-G-H-T; **et**
(c) H-Y-[TS]-X-D-[VI]-X(3)-[FYI]-X(6)-F-X(2)-Y-H
ou d'une séquence nucléotidique antisens de celle-ci pour identifier ou développer des composés influençant la réaction
CER + PE ↔ EPC + DAG
*in vitro.*

20. Procédé pour produire de la phosphatidyl:glycoprotéine cholinephosphotransférase ou de la phosphatidyl:glycolipide cholinephosphotransférase comprenant l'expression d'une séquence nucléotidique correspondante codant un polypeptide comprenant les motifs d'aminoacides :
(a) P-L-X-D-X(35-75)-R-R-X(8)-[YF]-X(2)-R-X(6)-T;
(b) C-X-D-X(3)-S-G-H-T; **et**
(c) H-Y-[TS]-X-D-[VI]-X(3)-[FYI]-X(6)-F-X(2)-Y-H
dans un (micro)organisme ou une lignée cellulaire selon la revendication 11 et l'isolement de la phosphatidyl:glycoprotéine cholinephosphotransférase ou de la phosphatidyl:glycolipide cholinephosphotransférase.

21. Procédé pour produire une glycoprotéine phosphorylcholine-substituée ou un glycolipide phosphorylcholine-substitué comprenant l'expression d'une séquence nucléotidique correspondante codant un polypeptide comprenant les motifs d'aminoacides :
(a) P-L-X-D-X(35-75)-R-R-X(8)-[YF]-X(2)-R-X(6)-T;
(b) C-X-D-X(3)-S-G-H-T; **et**
(c) H-Y-[TS]-X-D-[VI]-X(3)-[FYI]-X(6)-F-X(2)-Y-H
dans un (micro)organisme ou une cellule selon la revendication 11 et l'isolement de la glycoprotéine phosphorylcholine-substituée ou du glycolipide phosphorylcholine-substitué.

22. Utilisation d'une ou plusieurs des séquences nucléotidiques codant un polypeptide comprenant les motifs d'aminoacides :
(a) P-L-X-D-X(35-75)-R-R-X(8)-[YF]-X(2)-R-X(6)-T;
(b) C-X-D-X(3)-S-G-H.T; **et**
(c) H-Y-[TS]-X-D-[VI]-X(3)-[FYI]-X(6)-F-X(2)-Y-H
ou d'une séquence nucléotidique antisens qui est complémentaire de celle-ci pour influencer la réaction
glycolipide/protéine + PC <-> glycolipide/protéine PC-substitué + DAG
*in vitro.*

23. Utilisation d'une ou plusieurs des séquences nucléotidiques codant un polypeptide comprenant les motifs d'aminoacides :
(a) P-L-X-D-X(35-75)-R-R-X(8)-[YF]-X(2)-R-X(6)-T;
(b) C.X-D-X(3)-S-G-H-T; **et**
(c) H-Y-[TS)-X-D-[VI]-X(3)-[FYI]-X(6)-F-X(2)-Y-H
ou d'une séquence nucléotidique antisens qui est complémentaire de celle-ci pour identifier ou développer des composés influençant la réaction
glycolipide/protéine + PC <-> glycolipide/protéine PC-substitué + DAG
*in vitro.*

24. Procédé pour déterminer si un composé est capable d'abaisser ou de réguler positivement une activité enzymatique manifestée par une cellule, ladite activité comprenant une activité d'une enzyme du groupe d'enzymes identifiées comme étant des sphingomyéline synthases, des éthanolamine phosphorylcéramide synthases, des phosphatidylcholine:glycoprotéine cholinephosphotransférases et des phosphatidylcholine:glycolipide cholinephosphotransférases, ledit procédé comprenant la fourniture à ladite cellule d'un acide nucléique codant un polypeptide comprenant les motifs d'aminoacides :
(a) P-L-X-D-X(35-75)-R-R.X(8)-[YF]-X(2)-R-X(6)-T;
(b) C-X-D-X(3)-S-G-H-T; **et**
(c) H-Y-[TS]-X-D-[VI]-X(3)-[FYT]-X(6)-F-X(2)-Y-H
la mise en contact de ladite cellule avec ledit composé et la détermination de ce que ladite activité enzymatique est abaissée ou régulée positivement.

25. Procédé selon la revendication 24 où ladite cellule est déficiente en activité sphingomyéline synthase.

26. Procédé selon la revendication 24 ou la revendication 25, où ladite cellule est une cellule d'un microorganisme eucaryote.

27. Procédé selon la revendication 26, où ladite cellule est une cellule de levure.

28. Procédé selon l'une quelconque des revendications 24-27, où ledit polypeptide comprend une séquence d'aminoacides ayant au moins 90 % d'identité de séquence avec l'une quelconque de SEQ ID No. 12-22, comme représenté sur la figure 9.

29. Procédé selon l'une quelconque des revendications 24-28, où ledit polypeptide est dérivé d'un plasmodium.

30. Procédé selon l'une quelconque des revendications 24-29, où ledit composé comprend de l'ARN.

31. Utilisation d'un oligonucléotide spécifique d'une séquence d'acide nucléique codant un polypeptide comprenant les motifs d'aminoacides :
(a) P-L-X-D-X(35-75)-R-R-X(8)-[YF]-X(2)-RX(6)-T;
(b) C-X-D-X(3)-S-G-H-T; **et**
(c) H-Y-[TS]-X-D-[VI]-X(3)-[FYI]-X(6)-F-X(2)-Y-H
pour détecter ladite séquence,
où le polypeptide ne comprend pas de séquence d'aminoacides choisie parmi SEQ ID No. 12, 13, 15, 16, 19, 21 et 22 comme représenté sur la figure 9.

32. Utilisation selon la revendication 31, pour déterminer si une cellule comprend une activité sphingomyéline synthase.

33. Inhibiteur d'un polypeptide ayant une sphingomyéline synthase comprenant les motifs d'aminoacides :
(a) P-L-X.D.X(35-75)-R-R-X(8)-[YF]-X(2)-R-X(6).T;
(b) C-X-D-X(3)-S-G-H-T; **et**
(c) H-Y-[TS]-X-D-[VI]-X(3)-[FYI]-X(6)-F-X(2)-Y-H
destiné à être utilisé comme promoteur de la mort cellulaire.

34. Inhibiteur d'un polypeptide ayant une activité sphingomyéline synthase comprenant les motifs d'aminoacides :
(a) P-L-X-D-X(35-75)-R-R-X(8)-[YF]-X(2)-R-X(6)-T;
(b) C-X-D-X(3)-S-G-H-T; **et**
(c) H-Y-[TS]-X-D-[VI]-X(3)-[FYI]-X(6)-F-X(2)-Y-H
destiné à être utilisé pour favoriser la mort cellulaire.

35. Inhibiteur selon la revendication 33 ou 34, où ladite cellule est une cellule d'un parasite.

36. Inhibiteur selon la revendication 33 ou 34, où ladite cellule est une cellule humaine, de préférence une cellule tumorale.

37. Acide nucléique codant un polypeptide comprenant les motifs d'aminoacides :
(a) P-L-X-D-X(35-75)-R-R-X(8)-[YF]-X(2)-R-X(6)-T;
(b) C-X-D-X(3)-S-G-H-T; **et**
(c) H-Y-[TS]-X-D-[VI]-X(3)-[FYI]-X(6)-F-X(2)-Y-H
destiné à être utilisé pour augmenter la survie cellulaire et/ou la croissance cellulaire.

38. Procédé pour améliorer le rendement d'un produit de sécrétion d'une cellule comprenant la fourniture à ladite cellule d'un polypeptide comprenant les motifs d'aminoacides :
(a) P-L-X-D-X(35-75)-R-R-X(8)-[YF]-X(2)-R-X(6)-T;
(b) C-X-D-X(3)-S-G-H-T; **et**
(c) H-Y-[TS]-X-D-[VI]-X(3)-[FYI]-X(6)-F-X(2)-Y-H
ou d'un acide nucléique codant ledit polypeptide.

39. Procédé selon la revendication 38, comprenant la fourniture à ladite cellule d'un polypeptide comprenant une séquence d'aminoacides ayant au moins 90 % d'identité de séquence avec l'une quelconque de SEQ ID No. 12-22, comme représenté sur la figure 9, ou d'un acide nucléique codant un polypeptide comprenant une séquence d'aminoacides ayant au moins 90 % d'identité de séquence avec l'une quelconque de SEQ ID No. 12-22, comme représenté sur la figure 9.

40. Procédé selon la revendication 38 ou 39, où ladite cellule est une cellule d'un microorganisme eucaryote.

41. Procédé selon l'une quelconque des revendications 24-30 comprenant en outre la fourniture à ladite cellule ou à une fraction de celle-ci d'un substrat marqué pour ladite sphingomyéline synthase.

42. Procédé selon la revendication 41, comprenant en outre la récolte de sphingolipide à partir de ladite cellule ou de ladite fraction et la détection de sphingolipide marqué.

43. Procédé selon la revendication 41 ou 42, comprenant en outre la détection dudit sphingolipide marqué au moyen de chromatographie (sur couche mince) ou de spectrométrie de masse.

44. Procédé pour cibler un premier polypeptide comprenant les motifs d'aminoacides :
(a) P-L-X-D-X(35-75)-R-R-X(8)-[YF]-X(2)-R-X(6)-T;
(b) C-X-D-X(3)-S-G-H-T; **et**
(c) H-Y-[TS]-X-D-[VI]-X(3)-[FYI]-X(6)-F-X(2)-Y-H
vers un compartiment cellulaire différent comprenant la fourniture à une partie cytosolique dudit premier polypeptide d'un signal de localisation de compartiment cellulaire d'une partie cytosolique d'un second polypeptide comprenant les motifs d'aminoacides :
(a) P-L-X-D-X(35-75)-R-R-X(8)-[YF]-X(2)-R-X(6)-T;
(b) C-X-D-X(3)-S-G-H-T; **et**
(c) H-Y-[TS]-X-D-[VI]-X(3)-[FYI]-X(6)-F-X(2)-Y-H
où ledit premier et ledit second polypeptides, quand ils sont non modifiés, résident dans des compartiments cellulaires différents.

45. Procédé selon la revendication 44, où ladite partie cytosolique dudit premier polypeptide comprend l'extrémité C-terminale dudit polypeptide.

46. Procédé selon la revendication 44 ou la revendication 45, où ladite partie cytosolique dudit second polypeptide comprend l'extrémité C-terminale dudit polypeptide.

47. Procédé selon l'une quelconque des revendications 44-46, où lesdits compartiments cellulaires comprennent la membrane plasmique, le compartiment endosomique, le corps de Golgi, le réticulum endoplasmique ou une combinaison de ceux-ci.

48. Procédé selon l'une quelconque des revendications 44-47, où ledit signal de localisation de compartiment cellulaire dudit second polypeptide remplace la partie cytosolique C-terminale dudit premier polypeptide.
